# EUROPEAN PATENT SPECIFICATION

(11) **EP 4 126 208 B1**
(45) Date of publication and mention of the grant of the patent: **25.12.2024**
(21) Application number: 21713706.6
(22) Date of filing: 26.03.2021
(51) Int. Cl.: A61N 2/00, A61N 2/02

(54) **A MAGNETIC FIELD EXPOSURE SYSTEM AND USES THEREOF**
MAGNETFELDEXPOSITIONSSYSTEM UND VERWENDUNGEN DAVON
SYSTÈME D'EXPOSITION AU CHAMP MAGNÉTIQUE ET SES UTILISATIONS

(30) Priority: 27.03.2020 EP 20166447
(43) Date of publication of application: 08.02.2023
(73) Proprietor: Universität Bern, 3012 Bern (CH)
(72) Inventor: MÜLLER, Eliane, J, 3008 Bern (CH); HARITON, William, 3008 Bern (CH); BRUNNER, Brigitte, 3008 Bern (CH)
(74) Representative: HGF
(86) International application number: PCT/EP2021/058009
(87) International publication number: WO 2021/191443

(56) References cited:
- DE-A1- 2 655 723
- US-A1- 2016 106 996
- US-B1- 6 461 289

## Description

This invention relates to a magnetic field exposure system for use in exposing organic cells, cellular tissue or at least a part of a subject to a modulated low frequency magnetic field (LF-MF).

The invention further relates to a method for enhancing *in vitro* proliferation of cells, particularly human epidermal stem and progenitor cells and other primary cells by exposing them to said LF-MF.

### BACKGROUND

It is known that electric signals drive biochemical signals in the body's healing processes (Zhao et al, Nature 442: 457-460, 2006). It has been shown that nerve sprouting, the direction of nerve growth and the rate of epithelial wound healing are controlled co-ordinately by a wound-induced electric field (Song et al, Journal of Cell Science 117, 4681-4690, 2004; doi:10.1242/jcs.01341). Moreover, application of pulsed electric fields has been found to promote skin rejuvenation by inducing prominent proliferation of epidermal keratinocytes, formation of microvasculature, and secretion of collagen (Golberg et al, Nature Scientific Reports, 5:10187, 2014).

The downside of electrical treatments is that electric fields fail to penetrate deeply into the skin and body when applied in a non-invasive manner. According to Faraday's law and Maxwell's equation, electric fields are coupled with magnetic fields which in the lower range of the electromagnetic spectrum penetrate unhindered into the tissue. A permanent magnet produces a static magnetic field whereas a time-varied magnetic field is produced by passing an alternating current (AC) through a coil or wire. Unlike a static magnetic fields, a time-varied electromagnetic field induces a voltage difference in tissues which induces ion to flow. Hence, time-varying electromagnetic fields may be used to circumvent the short-comings of pure electric fields and pure magnetic fields, and the use of time-varying electromagnetic fields (EMF) has been suggested for various medical applications.

There are a variety of frequency definitions used in this field. International Commission on Non-Ionizing Radiation Protection (ICNIRP) defines time-varying electromagnetic fields (EMFs) and magnetic fields (MFs) with frequencies within the range of 1 Hz to 100 kHz as low frequency (electro)magnetic fields and (electro)magnetic fields (EMF/MF) with frequencies within the range of 100 kHz to 300 GHz Hz as high frequency (electro)magnetic fields. International Telecommunication Union (ITU) defines time-varying electromagnetic fields (EMFs) and magnetic fields (MFs) with frequencies within the range of 3 Hz to 30 Hz as extremely low frequency, with frequencies within the range of 30 Hz to 300 Hz as super-low frequency, with frequencies within the range of 300 Hz to 3 kHz as ultra-low frequency, with frequencies within the range of 3 to 30 kHz as very low frequency and with frequencies within the range of 30 to 300 kHz as low frequency EMF/MF.

As used herein, the term "extremely low frequency" (ELF) refers to (electro)magnetic fields with frequencies within the range of 1 Hz to 300 Hz.

As used herein, the term "low frequency" (LF) refers to (electro)magnetic fields with frequencies within the range of 300 Hz to 100 kHz.

Certain low frequency sound waves are considered potentially beneficial for wellbeing due to their cardiac and cerebral effects. For example, music tuned to a frequency of 432 Hz decreases the heart rate in humans more than 440 Hz tuned music (Calamassi et al, Explore, vol. 15, issue 4, pp. 283-290, July-August 2019; doi: 10.1016/j.explore.2019.04.001). 432 Hz tuned music has also been shown to have a significant calming effect as reflected by increased alpha-band brain activity without any significant effect upon the sleep latency in daytime naps (Dubey et al, "Effect of music of specific frequency upon the sleep architecture and electroencephalographic pattern of individuals with delayed sleep latency: A daytime nap study", Journal of Family Medicine and Primary Care, 2019; 8:3915-9).

ELF-EMF also enhances human bone marrow stem/progenitor cell differentiation (Maziarz et al. Stem Cell Research & Therapy, 7:54, 2016, doi: 10.1186/s13287-016-0312-5). Moreover, the specific EMF parameters frequency, intensity and time of exposure have been suggested to influence human bone marrow stem/progenitor cell differentiation in vitro (Ross et al, Stem Cell Res., July, 15(1): 96-108, 2015, doi:10.1016/j.scr.2015.04.009). ELF-EMF have also been suggested to promote skin wound healing. EMF is believed to have an anti-inflammatory effect by the modulation of cytokine profiles that drive the transition from a chronic pro-inflammatory state to an anti-inflammatory state of the healing process (Madduri et al, Defence Life Science Journal, Vol. 3, No. 3, pp. 293-300, July, 2018, doi: 10.14429/dlsj.3.12032; and Rosado et al, Front. Public Health, 26 March 2018; doi https://doi.org/10.3389/fpubh.2018.00085. Vianale et al (Br. J. Dermatol. 158(6), 1189-1196, 2008, doi: 10.1111/j.1365-2133.2008.08540.x) showed that ELF-EMF exposure (1 mT, 50 Hz, 48 h) increased the proliferative activity of the transformed human keratinocyte cell line HaCaT with no difference in cell viabilities detected.

Makarov et al ("Design and analysis of a whole body non-contact electromagnetic stimulation device with field modulation", doi: https://doi.org/10.1101/416065, September 2018) describes a whole-body, non-contact electromagnetic stimulation device based on the concept of a conventional MRI Radio Frequency (RF) resonating coil at a resonant (carrier) frequency of 100 kHz-150 kHz and a field modulation (amplitude modulation) option of 0.5-100 Hz and with an input power of up to 3 kW. A potential clinical application of the device is suggested to be treatment of chronic pain.

The frequency 7.83 Hz corresponds to a naturally occurring geomagnetic field known as the Schumann resonance. This frequency is believed to have a beneficial effect on human health (Guerriero, F. et al., Extremely low frequency electromagnetic fields stimulation modulates autoimmunity and immune responses: a possible immuno-modulatory therapeutic effect in neurodegenerative diseases; Neural Regen Res 11, 1888-1895 (2016)). A Nasa-sponsored study (NASA grant NSG-259-62) demonstrated that this frequency, whether naturally or artificially generated, regulates the human circadian clock (Wever, R., Influence of weak electromagnetic fields on the circadian periodicity of humans; Die Naturwissenschaften 55, 29-32 (1968)). Several other studies demonstrate that the brain activity of individuals exposed to this frequency are entrained to adapt to a similar frequency range, the alpha-band frequency of 8 - 13 Hz, thereby producing profound physiological effects (Salansky, N. et al., Responses of the nervous system to low frequency stimulation and EEG rhythms: clinical implications. Neurosci Biobehav Rev 22, 395-409 (1998); Wang, C.X. et al., Transduction of the Geomagnetic Field as Evidenced from alpha-Band Activity in the Human Brain; eNeuro 6 (2019)).

Alpha-band brain waves are known to reduce psychological stress and support a relaxed mood state, further increasing attention and memory (Cherry, N.J. Human intelligence: the brain, an electromagnetic system synchronised by the Schumann Resonance signal. Med Hypotheses 60, 843-844 (2003); and Wang, C.X. et al. supra). These brain waves also increase serotonin production and reduce cortisol levels, boosting the immune system (Guerriero, supra). Reduced psychological stress has been shown to enhance regenerative processes such as wound healing most likely through a beneficial effect on the immune system resulting from reduced stress levels (Britteon, P. et. al., Association between psychological health and wound complications after surgery. Br. J Surg. 104, 769-776 (2017)).

Enhancing serotonin production and reducing stress through cortisol suppression improves well being (Kraus, C., Castren, E., Kasper, S., Lanzenberger, R. "Serotonin and neuroplasticity - Links between molecular, functional and structural pathophysiology in depression" 10.1016/j.neubiorev.2017.03.007, March 2017). In rats, 10 Hz treatment at 690-720 µT intensity for 3 hours daily over 15 days induced an increase in serotonin in the raphe nuclei (Shahbazi-Gahrouei, D., Shiri, L., Alaei, H., Naghdi, N., "The effect of continuous ELF-MFs on the level of 5-HIAA in the raphe nucleus of the rat", 10.1093/jrr/rrv093, January 2016).

US 2003/231126 A1 relates to an apparatus for producing a natural electromagnetic alternating field, in the range of a few 100 Hz up to 20 kHz, close to a user's body to compensate for electrical stress acting on a user. The apparatus comprises an alternating field production means and an alternating field transmission means. These two means may be accommodated in a common housing, which for example may be worn immediately against the body of a user on a piece of clothing or integrated in a piece of furniture, such as a chair. It is also suggested to mix the natural electromagnetic alternating field with artificial electromagnetic alternating fields, for example, a very low frequency signal, such as a broadband pulse in the frequency range from 0.01 to 40 Hz.

WO 2008/127011 A2 relates to a low frequency magnetic field physical therapy apparatus consisting of magnetic field inducing coils, a pulse signal controller and a magnetic field inducing coil driving part. The apparatus may be manufactured in the form of a mattress. The apparatus further includes a frequency modulation program which modulates input frequencies (six brain waves, 12.5 Hz, 16.3 Hz, 23.4 Hz, 39.6 Hz, 87 Hz, 250 Hz, and Schumann resonance wave specified as being 8.7 Hz) to generate a number of pulse magnetic signals under 100 µT through magnetic field inducing coils.

WO 2013/139915 A1 relates to a method and a system for subjecting organic cells in a region of a subject to an extremely low frequency magnetic field. A generator produces a sinusoidal non-harmonic current signal having a predetermined frequency of between 7.5 Hz and 7.9 Hz and electromagnetic radiation of substantially between 0.7 mT and 3 mT. A resonance medium is operably connected to the generator and is energised by the signal and located adjacent organic cellular tissue in the region for a predetermined period, whereby organic cells in the region are subjected to a constant magnetic field of less than 1 mT and having a frequency of substantially between 7.5 Hz and 7.9 Hz for the predetermined period. At least one resonance medium may be mounted on a support medium selected from the group comprising a belt-like member, a mask-like member, a dressing, a mattress, a pillow, a helmet. Oral keratinocyte stem cells were stimulated with the apparatus of the invention for 7 days, i.e. subjected to a constant non-deformed ELF magnetic field of 7.692 Hz and 0.75 mT, and cellular development was assessed at 3 and 7 days. The system is suggested for use in regenerating cellular tissue composed of cells having keratin therein and for proliferating progenitor non-differentiated cells or stem cells in cellular tissue.

DE 26 55 723 discloses an apparatus which generates a pulsed magnetic field with a fundamental frequency of 30 to 100 Hz, a superimposed oscillation of 300 to 600 Hz and is clocked in frequency packages in the range of 1 to 30 Hz. The apparatus is stated to provide health-promoting effects in subjects exposed to the magnetic field.

US 2012/0203131 discloses an EEG device for the treatment of tinnitus which generates a low-frequency electromagnetic field with a frequency of 1 to 100 Hz and a magnetic field strength of less than 20 mT.

US 6,461,289 discloses a device which generates a magnetic field with a static component and an alternating component pulsed magnetic field.

There remains a need for magnetic field therapy that promotes beneficial effects in organic cells, particularly stem or progenitor cells (e.g. in tissue homeostasis or regeneration or *ex-vivo* cell culture) and/or systems that use magnetic fields to promotes well-being (e.g. reducing psychological stress) in subjects exposed to the magnetic field.

### BRIEF SUMMARY OF THE DISCLOSURE

An objective of the present invention is to provide an improved magnetic field (MF) exposure system for use in exposing organic cells (*in vitro*)*,* cellular tissue (*in vitro* or *in vivo*) or at least a part of a subject (e.g. a human) to an amplitude modulated low frequency magnetic field (LF-MF). Exposing cells (e.g. stem or progenitor cells) to the MF generated by the MF exposure system disclosed herein significantly improves cell survival rate, and/or increases cell proliferation, and/or reduces cellular stress, and/or reduces cell aging particularly after long term exposure, such as from 1 to 10 weeks (e.g. from 1 to 4 weeks).

Another object of the invention is the provision of a modulated low frequency magnetic field (LF-MF) exposure system for use in reducing stress, enhancing concentration, reducing anxiety, promoting well-being in a subject exposed to the LF-MF by, for example, inducing increased alpha brain wave activity and strengthening the immune system.

In a first aspect of the present invention, there is provided a MF exposure system for exposing organic cells, cellular tissue, or at least a part of a subject (e.g. a human) to a LF-MF, said system comprising a MF generator with one or more coils configured to produce a MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

Suitably the MF varies according to an amplitude-modulated signal with a carrier frequency within the range of 400 to 450 Hz, preferably about 432 Hz.

In certain embodiments the modulation frequency is less than 50 Hz, for example the modulation frequency is less than 30 Hz. Suitably the modulation frequency is from 0.5 to 50 Hz, e.g. from 0.5 to 30 Hz, from 0.5 to less than 30 Hz, from 3 to 30 Hz, from 3 to less than 30 Hz, or from 3 to 28 Hz. Preferably the modulation frequency corresponds to a Schumann resonance mode, particularly about 7.83 Hz. Suitably the MF field strength is from 5 to 200 µT, for example from 10 to 200 µT. Preferably the MF field strength is about 200 µT or less, for example about 100 µT, about 30 µT, or about 11 µT. Suitably the LF-MF produced by the system is a continuous magnetic field (i.e. the LF-MF is not clocked or pulsed).

The term "modulation frequency" as used herein may also be referred to as "amplitude variation frequency". The amplitude variation waveform is preferably a sinusoidal amplitude variation waveform. In use the coil or coils are supplied with a time-varying current that varies according to an amplitude-modulated signal, in which case the MF strength at a given point in space varies in proportion to the instantaneous current value. The MF exposure system of the invention may be used to expose organic cells, cellular tissue of at least a part of a subject (e.g. a human) to said LF-MF for any of the uses and methods described herein, for example promoting cellular regeneration in the cellular tissue. Particularly, the MF exposure system may be used to expose skin tissue of a human to said LF-MF for promoting cellular regeneration and/or tissue homeostasis. The system may be adapted to provide therapeutic (including curative, symptom-reducing or prophylactic) or non-therapeutic (e.g. cosmetic) treatment further described hereinafter It is noted that therapeutic methods are not a part of the invention.

Also disclosed is a MF exposure system for use in exposing organic cells, cellular tissue or at least a part of a subject (e.g. a human) to a LF-MF, said system comprising a MF generator with one or more coils configured to produce a MF that varies according to an amplitude-modulated signal with a carrier frequency within the range of 400 to 450 Hz, preferably 432 Hz, and wherein the MF has a MF strength within the range of 10 to 200 µT, preferably 200 µT. More particularly, the MF varies according to an amplitude-modulated signal with a carrier frequency within the range of 400 to 450 Hz, preferably 432 Hz, a modulation frequency within the range from 3 to 30 Hz, such as a modulation frequency corresponding to a Schumann resonance mode, preferably 7.83 Hz, and MF strength within the range of 10 to 200 µT, preferably 200 µT.

The Schumann resonance represents an electromagnetic phenomenon in the earth's atmosphere related to global lightning activity. Naturally occurring Schumann resonance is not measurable in presence of "electromagnetic smog" from electronic devices and is attenuated by buildings of concrete and iron. In 1952, Winfried Otto Schumann calculated that the resonances should occur at frequencies of 7.49[n(n+1)]^{1/2} Hz, wherein n is an integer. However, when measured by several observatories around earth (e.g. in Tomsk), the actual Schumann frequency modes differ slightly from this calculation with the fundamental Schumann resonance at approximately 7.83 Hz and with higher harmonics occurring at approximately 14.1, 20.3, 26.4, and 32.4 Hz, etc. However, there are geographical, diurnal and seasonal variations of the different modes of the Schumann resonance.

It has surprisingly been found that exposure of stem and progenitor cells (e.g. human stem and progenitor cells, particularly human epidermal keratinocytes) to a LF-MF that varies according to an amplitude-modulated signal with a carrier frequency of 432 Hz, a sinusoidal modulation frequency of 7.83 Hz, and a MF strength of 200 µT significantly improves cell survival, and/or increases cell proliferation, and/or reduces cell aging, and/or reduces cell stress when compared to exposure to a MF of 7.83 Hz at 200 µT alone or to a non-modulated LF-MF of 432 Hz at 200 µT. Enhancing the body's ability to efficiently recruit its own stem cells, instead of multiplying cells which have already been copied many times while accumulating DNA mutations and other damages, represents a powerful means of regenerative tissue homeostasis abating chronic dysfunctions and diseases including cancer.

As illustrated in the Examples, the modulated LF-MF described herein is particularly effective for enhancing *in-vitro* proliferation of stem and progenitor (e.g. , keratinocyte stem or progenitor cells, particularly human keratinocyte stem or progenitor cells). The system may therefore be configured to provide a cell culture system which in use increases cell proliferation, and/or cell viability, and/or reducing cell stress, thereby providing improved cell culture/population expansion by exposing the cells to the modulated LF-MF.

Accordingly, in an embodiment there is provided a MF exposure cell culture system, said system comprising a cell culture vessel defining a cell culture cavity within the vessel and a MF generator with one or more coils configured to produce a MF within the cell culture cavity that varies according to an amplitude-modulated signal with a carrier frequency of from 360 to 450 Hz, wherein the modulation frequency is from 0.5 to 100 Hz; and wherein the MF has a field strength of from 0.5 to 250 µT.

In a further embodiment the MF exposure cell culture system further comprises one or more organic cells (preferably a population of stem or progenitor cells, or other primary cell) in the cell culture cavity. Suitably wherein the cell culture cavity further comprises a cell culture medium. In use the MF exposure cell culture system, exposes cells in the cell culture vessel to the modulated LF-MF described herein, thereby increasing cell proliferation, reducing cell stress, improving cell viability and/or inhibiting cellular senescence.

In a second aspect, there is provided a method for improving wellbeing, reducing stress (e.g. psychological stress), enhancing concentration and/or reducing anxiety in a subject by exposing at least the brain of the subject (e.g. a healthy human), to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

Suitably the MF varies according to an amplitude-modulated signal with a carrier frequency within the range of 400 to 450 Hz, preferably about 432 Hz. It may be that the modulation frequency is less than 50 Hz, preferably the modulation frequency is less than 30 Hz. Suitably the modulation frequency is from 0.5 to 50 Hz, e.g. from 0.5 to 30 Hz, from 0.5 to less than 30 Hz, from 3 to 30 Hz, or from 3 to 28 Hz. Preferably the modulation frequency corresponds to a Schumann resonance mode, more preferably about 7.83 Hz. Suitably the MF field strength is from 0.5 to 200 µT, for example from 5 to 200 µT, example from 10 to 200 µT, from 10 to 100 µT, from 10 to 50 µT, from 10 to 50 µT, from 0.5 to 40 µT, from 0.5 to 15 µT or from 0.5 to 12 µT. Thus it may be that the MF field strength is about 200 µT, about 100 µT, about 30 µT or about 11 µT.

Also disclosed is a non-therapeutic method for improving wellbeing, reducing stress (e.g. psychological stress), enhancing concentration and/or reducing anxiety in a subject by exposing at least a part of the subject, particularly a healthy human, to a LF-MF that varies according to an amplitude-modulated signal with a carrier frequency within the range of 400 to 450 Hz, preferably 432 Hz, and wherein the MF has a MF strength within the range of 10 to 200 µT, preferably 200 µT. More particularly, the MF varies according to an amplitude-modulated signal with a carrier frequency within the range of 400 to 450 Hz (preferably 432 Hz), a modulation frequency within the range of 3 to 30 Hz, such as a modulation frequency corresponding to a Schumann resonance mode (preferably 7.83 Hz), and a MF strength within the range of 10 to 200 µT (for example up to about 11 µT, up to about 30 µT or up to about 200 µT).

In a third aspect, there is provided the use of a LF-MF that varies according to an amplitude modulated signal with a carrier frequency of from 360 to 450 Hz, wherein the modulation frequency is from 0.5 to 100 Hz; and wherein the MF has a field strength of from 0.5 to 250 µT, for improving wellbeing, reducing stress (e.g. psychological stress), enhancing concentration and/or reducing anxiety in a subject (e.g. a healthy human), by exposing at least the brain of the subject to the LF-MF. Suitably the MF varies according to an amplitude-modulated signal with a carrier frequency within the range of 400 to 450 Hz, preferably about 432 Hz. It may be that the modulation frequency is less than 50 Hz, preferably the modulation frequency is less than 30 Hz. Suitably the modulation frequency is from 0.5 to 50 Hz, e.g. from 0.5 to 30 Hz, from 0.5 to less than 30 Hz, from 3 to 30 Hz, or from 3 to 28 Hz. Preferably the modulation frequency corresponds to a Schumann resonance mode, more preferably about 7.83 Hz. Suitably the MF field strength is from 5 to 200 µT, for example from 10 to 200 µT, from 10 to 100 µT, from 10 to 50 µT, from 10 to 50 µT. Thus it may be that the MF field strength is about 200 µT, about 100 µT, about 30 µT, or about 11 µT.

Also disclosed herein is a non-therapeutic use of a LF-MF that varies according to an amplitude modulated signal with a carrier frequency within the range of 400 to 450 Hz (preferably 432 Hz) and a MF strength within the range of 10 to 200 µT (preferably 200 µT), for improving wellbeing, reducing stress (e.g. psychological stress), enhancing concentration and/or reducing anxiety in a subject, particularly a healthy human, by exposing at least a part of the subject to the LF-MF. More particularly, the MF varies according to an amplitude-modulated signal with a carrier frequency within the range of 400 to 450 Hz (preferably 432 Hz), a modulation frequency within the range of 3 to 30 Hz, such as a modulation frequency corresponding to a Schumann resonance mode (preferably 7.83 Hz), and a MF strength within the range of 0.5 to 250 µT( e.g. 0.5 to 200 µT, or 10 to 200 µT (preferably 200 µT)).

The second and third aspects relate to non-therapeutic methods.

In the second and third aspects the subject may, for example, be exposed to the modulated LF-MF for up to 8 hours (e.g. up to 8 hours, up to 5 hours, up to 4 hours up to 3 hours, up to 2 hours or up to 1 hour per day. Thus is may be that the subject is exposed to the LF-MF for 10 minutes to 1 hour per day during a time period of 1-8 weeks, such as 4 weeks. Specifically, the MF varies according to an amplitude-modulated signal with a carrier frequency of from 360 to 450 Hz (e.g. within the range of 400 to 450 Hz (preferably about 432 Hz), a modulation frequency within the range of 3 to 30 Hz (e.g. from 0.5 to less than 30 Hz), such as a modulation frequency corresponding to a Schumann resonance mode (preferably about 7.83 Hz), and MF strength of 0.5 to 250 µT( e.g. 0.5 to 200 µT, or 10 to 200 µT (e.g. within the range of 10 to 200 µT)).

In a fourth aspect, there is provided a cosmetic method for treating a condition associated with skin stem cells or skin progenitor cells in a subject, (e.g. a healthy human), the method comprising exposing cellular tissue of at least a part of the subject to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

In a fifth aspect, there is provided a non-therapeutic use of a LF-MF that varies according to an amplitude modulated signal with a carrier frequency of from 360 to 450 Hz, wherein the modulation frequency is from 0.5 to 100 Hz; and wherein the MF has a field strength of from 0.5 to 250 µT, for treating a condition associated with skin stem cells or skin progenitor cells in a subject (e.g. a healthy human), by exposing cellular tissue of at least a part of the subject to the LF-MF.

The condition associated with skin stem cells or skin progenitor cells in the fourth and fifth aspects may, for example, be associated with skin stem cells selected from epidermal stem cells, melanocyte stem cells, hair follicle stem cells, keratinocyte stem, cells and keratinocyte progenitor cells or fibroblasts. Preferably the condition is associated with keratinocyte stem cells or keratinocyte progenitor cells.

The non-therapeutic (cosmetic) use in the fourth and fifth aspect may, for example, be selected from: maintaining skin homeostasis, reducing skin aging, enhancing skin rejuvenation, preventing or reducing skin wrinkles, increasing skin elasticity, preventing or reducing skin stretch marks, preventing or reducing cellulitis, increasing skin hydration, reducing skin roughness, reducing skin pore size, inducing hair growth, preventing or reducing hair loss, inhibiting hair greying, inducing hair re-pigmentation, maintaining skin pigmentation, and inducing nail growth. Thus it may be that the use is inducing hair growth. It may be that the use is preventing or reducing hair loss. It may be that the use is reducing skin aging.

Suitably in the fourth and fifth aspects the MF varies according to an amplitude-modulated signal with a carrier frequency within the range of 400 to 450 Hz, preferably about 432 Hz. It may be that the modulation frequency is less than 50 Hz, preferably the modulation frequency is less than 30 Hz. Suitably the modulation frequency is within the range from 3 to 28 Hz. Suitably the modulation frequency corresponds to a Schumann resonance mode, preferably about 7.83 Hz. Suitably the MF field strength is from 5 to 200 µT, for example from 10 to 200 µT. Preferably the MF field strength is about 200 µT.

Also disclosed is a cosmetic method for reducing skin aging (e.g. by enhancing skin rejuvenation, reducing wrinkles, increasing skin elasticity, reducing stretch marks and/or reducing cellulitis) in a subject, particularly a healthy human, by exposing cellular tissue of at least a part of the subject to LF-MF that varies according to an amplitude-modulated signal with a carrier frequency within the range of 400 to 450 Hz (preferably 432 Hz) and a MF strength within the range of 0.5 to 250 µT( e.g. 0.5 to 200 µT, or 10 to 200 µT, preferably 200 µT).

Reduced skin aging is believed to be achieved through stem cell activation, such as enhanced proliferation of human stem and progenitor keratinocytes. More particularly, the MF varies according to an amplitude-modulated signal with a carrier frequency within the range of 400 to 450 Hz, a modulation frequency within the range of 3 to 30 Hz, such as a modulation frequency corresponding to a Schumann resonance mode (preferably 7.83 Hz), and MF strength within the range of 0.5 to 250 µT( e.g. 0.5 to 200 µT, or 10 to 200 µT.

Also disclosed herein is a non-therapeutic, cosmetic use of a LF-MF that varies according to an amplitude-modulated signal with a carrier frequency within the range of 400 to 450 Hz (preferably 432 Hz) and a MF strength within the range of 10 to 200 µT (preferably 200 µT), for reducing skin aging (e.g. by enhancing skin rejuvenation, reducing wrinkles, increasing skin elasticity, reducing stretch marks and/or reducing cellulitis) in a subject, particularly a healthy human, by exposing cellular tissue of at least a part of the subject (e.g. the facial tissue of the subject) to the LF-MF. For example, cellular tissue of at least a part of the subject may be exposed to the LF-MF for 10 minutes to 1 hour per day during a time period of 1-8 weeks, such as 4 weeks. More particularly, the MF varies according to an amplitude-modulated signal with a carrier frequency within the range of 400 to 450 Hz, a modulation frequency within the range of 3 to 30 Hz, such as a modulation frequency corresponding to a Schumann resonance mode (preferably 7.83 Hz), and MF strength within the range of 0.5 to 250 µT( e.g. 0.5 to 200 µT, or 10 to 200 µT).

In a sixth aspect there is provided a therapeutic non-claimed method for treating a medical condition associated with stem cells or progenitor cells in a subject (e.g. a human), by exposing at least a part of the subject to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

In a seventh aspect there is provided a therapeutic, non-claimed use of a LF-MF that varies according to an amplitude modulated signal with a carrier frequency of from 360 to 450 Hz, wherein the modulation frequency is from 0.5 to 100 Hz; and wherein the MF has a field strength of from 0.5 to 250 µT, for treating a medical condition associated with stem cells or progenitor cells in a subject (e.g. a human), by exposing at least a part of the subject to the LF-MF.

In the sixth and seventh aspects, the medical condition may be associated with adult stem cells (also referred to as somatic stem cells). For example, the medical condition may be associated with muscle stem cells, hematopoietic stem cells, epithelial stem cells, neural stem cells, mesenchymal stem cells, mammary stem cells, intestinal stem cells, mesodermal stem cells, endothelial stem cells, skin stem cells, olfactory stem cells, neural crest stem cells, dental pulp stem cells, or fibroblasts. In particular, the medical condition is associated with skin stem cells, for example keratinocyte stem cells, such as human epidermal keratinocyte stem cells.

In the sixth and seventh aspects, the medical condition may be tissue regeneration, wound healing, bone regeneration, inflammatory disease, cardiovascular disease, a neurodegenerative condition, a cognitive disorder, an autoimmune disease, osteoarthritis, tissue fibrosis, periodontal disease, or a dermatological disorder. In particular embodiments the medical condition is wound healing (including healing of chronic wounds), for example healing of surgical wounds, trauma wounds, abrasions, burns (including radiation burns, e.g. sunburn or burns resulting from radiotherapy), blisters, ulcers (including diabetic foot ulcers and venous leg ulcers) or preventing or minimising scarring (e.g. by preventing or inhibiting cutaneous fibrosis). In a further embodiment the LF-MF enhances tissue regeneration in the subject, for example regeneration of a tissue selected from muscle tissue (e.g. to maintain or increase muscle mass or to repair muscle), connective tissue, joint tissue, epithelial tissue, endothelial tissue, nerves, brain tissue, fat tissue, skin tissue, lung tissue, liver tissue, bladder tissue, kidney tissue, heart tissue, stomach tissue, intestinal tissue, spinal tissue, eye tissue, fibrous tissue, dentin, bone, and bone marrow.

In an eighth aspect, there is provided a therapeutic, non-claimed use of a LF-MF that varies according to an amplitude modulated signal with a carrier frequency within the range of 360 to 450 Hz (e.g. 400 to 450 Hz (preferably 432 Hz)) and a MF strength within the range of 0.5 to 250 µT (e.g.10 to 200 µT (preferably 200 µT)), for enhancing tissue regeneration (e.g. for post-operative tissue healing), enhancing wound healing, treating cancer and/or enhancing immune function in a subject in need thereof by exposing cellular tissue of at least a part of the subject to the LF-MF. For example, cellular tissue of at least a part of the subject may be exposed to the LF-MF for 10 min up to 24 hours per day during a time period of 1-8 weeks, such as 4 weeks. More particularly, the MF varies according to an amplitude-modulated signal with a carrier frequency within the range of 400 to 450 Hz, a modulation frequency within the range of 0.5 to 100 Hz (e.g. 3 to 30 Hz, or 3 to less than 30 Hz, such as a modulation frequency corresponding to a Schumann resonance mode (preferably 7.83 Hz)), and MF strength within the range of 0.5 to 250 µT( e.g. 0.5 to 200 µT, or 10 to 200 µT).

In a ninth aspect there is provided a method for enhancing tissue regeneration in a subject in need thereof by exposing cellular tissue of at least a part of the subject to a LF-MF that varies according to an amplitude-modulated signal with a carrier frequency within the range of 360 to 450 Hz (e.g. 400 to 450 Hz (preferably 432 Hz)) and a MF strength within the range of 0.5 to 250 µT (e.g.10 to 200 µT (preferably 200 µT)). Enhanced tissue regeneration is believed to be achieved through stem cell activation. More particularly, the MF varies according to an amplitude-modulated signal with a carrier frequency within the range of 400 to 450 Hz, a modulation frequency within the range of 0.5 to 100 Hz (e.g. 3 to 30 Hz, or 3 to less than 30 Hz), such as a modulation frequency corresponding to a Schumann resonance mode (preferably 7.83 Hz), and MF strength within the range of 0.5 to 250 µT( e.g. 0.5 to 200 µT, or 10 to 200 µT).

In a tenth aspect there is provided a method for enhancing wound healing in a subject in need thereof by exposing cellular tissue of at least a part of the subject to a LF-MF that varies according to an amplitude-modulated signal with a carrier frequency within the range of 360 to 450 Hz (e.g. 400 to 450 Hz (preferably 432 Hz)) and a MF strength within the range of 0.5 to 250 µT (e.g. 10 to 200 µT (preferably 200 µT)). Without wishing to be bound by theory, enhanced wound healing is believed to be achieved through stem cell activation. More particularly, the MF varies according to an amplitude-modulated signal with a carrier frequency within the range of 400 to 450 Hz, a modulation frequency within the range of 0.5 to 100 Hz (e.g. 3 to 30 Hz, or 3 to less than 30 Hz, such as a modulation frequency corresponding to a Schumann resonance mode (preferably 7.83 Hz), and MF strength within the range of 0.5 to 250 µT( e.g. 0.5 to 200 µT, or 10 to 200 µT).

In a eleventh aspect there is provided a method for treating, including preventing and/or reducing, cancer in a subject in need thereof by exposing cellular tissue of at least a part of the subject to a LF-MF that varies according to an amplitude-modulated signal with a carrier frequency within the range of 360 to 450 Hz (e.g. 400 to 450 Hz (preferably 432 Hz)) and a MF strength within the range of 0.5 to 250 µT (e.g.10 to 200 µT (preferably 200 µT)). Cancer prevention and reduction is believed to be achieved through stem cell activation. More particularly, the MF varies according to an amplitude-modulated signal with a carrier frequency within the range of 400 to 450 Hz, a modulation frequency within the range of 0.5 to 100 Hz (e.g. 3 to 30 Hz, or 3 to less than 30 Hz, such as a modulation frequency corresponding to a Schumann resonance mode (preferably 7.83 Hz)), and MF strength within the range of 0.5 to 250 µT( e.g. 0.5 to 200 µT, or 10 to 200 µT). In other embodiments the system and methods described herein are not for use in preventing and/or reducing, cancer in a subject.

In a twelfth aspect there is provided a method for enhancing the immune function in a subject in need thereof by exposing cellular tissue of at least a part of the subject to a LF-MF that varies according to an amplitude-modulated signal with a carrier frequency within the range of 360 to 450 Hz (e.g. 400 to 450 Hz (preferably 432 Hz)) and a MF strength within the range of 0.5 to 250 µT (e.g. 10 to 200 µT (preferably 200 µT)). Enhanced immune function is believed to be achieved through epidermal and mesenchymal stem cell activation. More particularly, the MF varies according to an amplitude-modulated signal with a carrier frequency within the range of 400 to 450 Hz, a modulation frequency within the range of 0.5 to 100 Hz (e.g. 3 to 30 Hz, or 3 to less than 30 Hz, such as a modulation frequency corresponding to a Schumann resonance mode (preferably 7.83 Hz)), and MF strength within the range of 10 to 200 µT.

In a thirteenth aspect there is provided a method for enhancing proliferation of organic cells, especially stem cells or progenitor cells, or other primary cells by exposing the cells (e.g. stem cells or progenitor cells) to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

When the cells are stem cells or progenitor cells, the cells may be any of the stem or progenitor cells disclosed herein, in particular human stem cells or human progenitor cells. The stem cells are suitably adult stem cells, although other stem cell types may also be used in the method, for example induced pluripotent stem cells, amniotic stem cells or embryonic stem cells. Examples of adult stem cells include, for example, muscle stem cells, hematopoietic stem cells, epithelial stem cells, neural stem cells, mesenchymal stem cells, mammary stem cells, intestinal stem cells, mesodermal stem cells, endothelial stem cells, skin stem cells, melanocyte stem cells, hair follicle stem cells, olfactory stem cells, neural crest stem cells and dental pulp stem cells. In particular the stem or progenitor cells are skin stem cells or skin progenitor cells, for example keratinocyte stem or progenitor cells. In other embodiments the stem cells are mesenchymal stem cells.

It may be that the method is an *in-vivo* method, wherein a subject is exposed to the LF-MF, thereby increasing proliferation of stem cells or progenitor cells in the subject. The increased stem cell proliferation is expected to enhance, for example, tissue regeneration in the subject. Suitably, the LF-MF is directed to a particular area of the body of the subject containing the stem cells, for example a body joint such as a knee or hip joint, an injury site on or in the body of the subject or an area of skin or hair on the subject. In a particular embodiment of the method a wound in the subject is exposed to the LF-MF to promote wound healing. For example the method may increase the rate of wound closure, and/or prevent or reduce the formation of scar tissue.

The method may be an *in-vitro* method, wherein a one or more organic cells (e.g. stem cells or progenitor cells, or other primary cells) are exposed to the LF-MF thereby increasing proliferation and expanding the population of the cells ( e.g. stem or progenitor cells) *in-vitro* (e.g. in a cell culture vessel). The *in-vitro* method may further comprise differentiating stem or progenitor cells into specific tissue cells, for example osteoblasts, fibroblasts (e.g. gingival fibroblasts), skin fibroblasts, keratinocytes, or chondrocytes. The method may also be used to generate particular tissues from the stem or progenitor cells, for example by differentiation of the stem or progenitor cells into skin, cartilage, ligaments, tendon or bone. The formation of specific tissues *in-vitro* may be performed by culturing the stem cells or progenitor cells in the presence of a suitable scaffold to promote tissue formation/structure. The scaffold may be, for example, a collagen or polymer scaffold or matrix. The cells may be exposed to the LF-MF during both the cell proliferation and differentiation stages of the method. Alternatively, the LF-MF may be applied only during the stem cell or progenitor cell proliferation, or only during cell differentiation.

The expanded stem cells or progenitor cells prepared according to the *in-vitro* method may be used in regenerative tissue therapies, for example by locally administering the cells to a site in a subject requiring cell or tissue regeneration. For example, the stem or progenitor cells prepared according to the method may be administered to muscle tissue, connective tissue, joint tissue, epithelial tissue, endothelial tissue, nervous tissue, fat tissue, skin tissue, lung tissue, liver tissue, bladder tissue, kidney tissue, cardiac tissue, pancreatic tissue, pancreatic tissue, stomach tissue, intestinal tissue, spinal tissue, brain tissue, eye tissue, fibrous tissue, dentin, bone or bone marrow. For example chondrocyte progenitor cells prepared according to the method may be injected into a knee of hip joint of a subject to promote cartilage regeneration or growth in the subject. Suitably the cells administered to the subject are autologous cells. However, administration of allogenic cells prepared according to the method are also contemplated.

In a further embodiment the method may be used to prepare cultured epidermal autografts *in-vitro,* the method comprising culturing skin stem cell or skin progenitor cells (preferably keratinocyte stem cells or keratinocyte progenitor cells and/or fibroblasts) wherein the stem or progenitor cells are exposed to the LF-MF to enhance proliferation and confluence of the cells to form an epithelial layer.

Culturing keratinocytes to form epithelial sheets may be carried out using known conditions, for example, as described in Hynds et al. (EMBO Mol Med. 2018 Feb; 10(2): 139-150) and Green et al., (Formation of epidermis by serially cultivated human epidermal cells transplanted as an epithelium to athymic mice; 1980, Transplantation 29: 308-313). Suitably the keratinocyte stem or progenitor cells are cultured on suitable substrate such as fibrin or a polymeric matrix (Pellegrini et al., (1999),Transplantation 68: 868-879 and Zhu et al., (2005), Eur J Plast. Surg. 28: 319-330).

The resulting epidermal autografts may be used to treat wounds, burns, ulcers (including diabetic ulcers, particularly diabetic foot ulcers) and the like.

A further embodiment provides a method for tissue regeneration in a subject comprising:
(i) culturing one or more stem cells or progenitor cells from the subject *in vitro,*
   wherein the cells are exposed to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT during at least part of the culturing, thereby expanding the population of stem or progenitor cells;
(ii) harvesting the cells; and
(iii) administering the cells to the subject.

In a further embodiment of this process after step (i) the expanded population of stem or progenitor cells from step (i) are differentiated into a target cell. The target cells are then harvested and administered to the subject according to steps (ii) and (iii) of the process. Differentiation of the stem or progenitor cells may be achieved by culturing the cells to confluence. Optionally the LF-MF is applied to the cells during differentiation.

In step (i) of the process the stem or progenitor cells are suitable exposed to the LF-MF throughout the culturing step. The stem cells or progenitor cells used in the process may be any of the stem or progenitor cells disclosed herein.

The *in-vitro* methods for enhancing proliferation of cells described herein is suitable for use with a broad range of organic cells, including stem cells, progenitor cells and other primary cells. Thus the method may be suitable for enhancing proliferation of primary cells *in vitro.* For example, primary cells selected from epithelial cells, endothelial cells, fibroblasts, melanocytes, keratinocytes, neurons, astrocytes, hepatocytes, skeletal muscle cells, smooth muscle cells, osteoblasts, myocytes, chondrocytes, adipocytes, synoviocytes, hair cells or blood cells. The Primary cells are cells that are obtained from the tissue of origin and are cultured i*n vitro* in the presence of the modulated LF-MF as described herein to enhance the proliferation and thus expand the population of cells. The primary cells obtained according to the method may be used in a variety of applications. For example to provide cells for use in cell-based assays for screening of drugs, toxicity assays, or studying cell biology.

Accordingly the culture of primary cells (or primary tissue culture) is of great interest, because primary cell cultures are generally considered more representative of *in-vivo* cells than immortalised cell lines. However, primary cells can be challenging to culture *in vitro,* because the cells have limited capacity to divide and often enter senescence after only a few cell cycles. This is particularly the case in primary cells that are terminally differentiated into a specific tissue type. As illustrated in the Examples herein it has been found that the modulated LF-MF described herein can inhibit cell senescence and may also provide other benefits for cell culture such as reduced cellular stress and/or enhance cell viability, and may therefore be beneficial for the culture of organic cells generally, especially the culture of stem cells, progenitor cells and other primary cells. Thus also provided is an *in-vitro* method for culturing organic cells by exposing the cells (e.g. stem cells or progenitor cells or other primary cells) to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

The *in-vitro* methods for culturing cells using the modulated LF-MF described herein may also be useful for the culture of cell lines, that is cells that have become immortalised. Cells can become immortalised spontaneously or as a result of genetic modification. Cell lines are well known and include but not limited to, for example, mammalian cell lines (e.g. HEK cells, HeLa cells, Namalwa cells, Chinese hamster ovary (CHO) cells and derivatives thereof (e.g. CHO-K1, CHO DG44 or CHO DXB11), baby hamster kidney (BHK-21) cells, 293 transformed kidney cells, WI-38 (human diploid normal embryonic lung cells), MRC-5 (human diploid normal embryonic lung cells), HepG2 (liver cancer transformed cell), myeloma cells (e.g. NS/O) or Vero cells. The *in-vitro* methods for culturing cells using the modulated LF-MF described herein may therefore also be used to enhance the culture and growth of cell-lines used in the manufacture of recombinant protein, viruses and vaccines.

In a further aspect of the present invention, there is provided a method for enhancing *in vitro* proliferation of human epidermal stem and progenitor cells by exposing the cells to a LF-MF that varies according to an amplitude-modulated signal with a carrier frequency within the range of 360 to 450 Hz (e.g. 400 to 450 Hz (preferably 432 Hz)) and a MF strength within the range of 0.5 to 250 µT (e.g. 10 to 200 µT (preferably 200 µT)). More particularly, the MF varies according to an amplitude-modulated signal with a carrier frequency within the range of 400 to 450 Hz, a modulation frequency within the range of 0.5 to 100 Hz (e.g. 3 to 30 Hz, or 3 to less than 30 Hz, such as a modulation frequency corresponding to a Schumann resonance mode (preferably 7.83 Hz)), and MF strength within the range of 0.5 to 250 µT( e.g. 0.5 to 200 µT, or 10 to 200 µT).

Disclosed herein is the use of a LF-MF that varies according to an amplitude-modulated signal with a carrier frequency within the range of 360 to 450 Hz (e.g. 400 to 450 Hz (preferably 432 Hz)) and a MF strength within the range of 0.5 to 250 µT (e.g. 10 to 200 µT (preferably 200 µT), for enhancing *in vitro* proliferation of human epidermal stem and progenitor cells by exposing the cells to the LF-MF. For example, the cells may be continuously exposed to the LF-MF during a time period of 1-8 weeks, such as 4 weeks. More particularly, the MF varies according to an amplitude-modulated signal with a carrier frequency within the range of 400 to 450 Hz and a modulation frequency within the range of 0.5 to 100 Hz (e.g. 3 to 30 Hz, or 3 to less than 30 Hz), such as a modulation frequency corresponding to a Schumann resonance mode (preferably 7.83 Hz), and MF strength within the range of 0.5 to 200 µT, e.g. 10 to 200 µT. The Examples herein show that exposing stem and progenitor cells to the LF-MF enhances cell proliferation, whilst also reducing cellular stress and/or minimising cellular aging. These features of the LF-MF are expected to be particularly beneficial for the *in-vitro* cell culture methods described herein, by, for example, maximising the proliferative potential of the cells cell, improving cell survival and/or improving cell viability during cell culture process.

It has been shown in the Examples herein that stem and progenitor cells exposed to the modulated LF-MF according to the invention exhibit a lower telomere length reduction following multiple cell divisions compared to cells that are not exposed to the modulated MF. Shortening of telomeres is associated with cell aging and results in cell senescence. Cell senescence and accumulation of senescent cells in the body is associated with a number of medical conditions, particularly age-related conditions (Zhao et al., Telomere length maintenance, shortening, and lengthening. J Cell Physiol. 229, 1323-1329 (2014)).

Accordingly, a further aspect provides a method for inhibiting cellular senescence comprising exposing cells to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

The cell may be a somatic cell. More particularly the cell is a stem cell or progenitor cell, for example any of the stem or progenitor cells, or other primary cells disclosed herein, preferably human stem or progenitor cells. Still more particularly the cell is a skin stem cell or skin progenitor cell, for example a keratinocyte stem cell or keratinocyte progenitor cell, and preferably a human keratinocyte stem cell or human keratinocyte progenitor cell.

In some embodiments the method for inhibiting cellular senescence is an *in vitro* method.

In some embodiments the method for inhibiting cellular senescence is an *in vivo* method carried out on a subject. When performed *in vivo* cellular tissue of at least a part of the subject is exposed to the LF-MF. For example the LF-MF may be directed to a particular region of the body, or targeted to an organ or tissue type of interest, for example the LF-MF may be directed to the head, torso, arms, legs, brain, eyes, liver, kidney, muscle, bone, skin, hair, brain, heart, stomach, intestine or colon.

In a further embodiment there is provided a method for treating a senescence associated disease in a subject, comprising exposing cellular tissue of at least a part of the subject to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

*In vivo* methods for treating senescence associated diseases are not claimed and are not a part of the invention.

The senescence associated disease may be an age related disease or condition. For example, the senescence associated disease may be selected from: a cardiovascular disorder (e.g. associated with arteriosclerosis (e.g. atherosclerosis)); a pulmonary disease (e.g. idiopathic pulmonary fibrosis, asthma, chronic obstructive pulmonary disease); osteoarthritis; a senescence-associated dermatological disease or disorder, a neurodegenerative disease (e.g. multiple sclerosis, Alzheimer's disease, Parkinson's disease, Huntington's disease or motor neurone disease), an ocular disease (e.g. age-related macular degeneration, glaucoma, diabetic retinopathy or cataracts), diabetes ( e.g. pancreatic diabetes), a liver disease (e.g. non-alcoholic steatohepatitis (NASH), primary biliary cholangitis (PBC), or primary sclerosing cholangitis (PSC)), sarcopenia and prostatic hyperplasia.

Guerrio et al., supra reviews the effects of low frequency electromagnetic fields on autoimmunity and immunomodulation, and various medical conditions, including neurodegenerative diseases. The review also indicates that low-frequency MF may be beneficial in the treatment of cognitive defects, psychiatric disorders, and anti-inflammatory effects in conditions such as rheumatoid arthritis. LF-MF are stated to have an effect on the activation of macrophages and may result in beneficial immunomodulation in subjects exposed to LF-MF.

Accordingly also provided is a system according to the first aspect for use in the treatment of a condition selected from an autoimmune disease, a neurodegenerative disease, an inflammatory disease, a cognitive disorder, and a psychiatric disorder.

Also provided is a method of treating a disorder selected from an autoimmune disease, a neurodegenerative disease, an inflammatory disease, a cognitive disorder, and a psychiatric disorder in a subject, the method comprising exposing at least a part of the subject to a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT.

Further provided is the use of a LF-MF that varies according to an amplitude-modulated signal with a carrier frequency of from 360 to 450 Hz, wherein the modulation frequency is from 0.5 to 100 Hz; and wherein the MF has a field strength of from 0.5 to 250 µT, for treating a disorder selected from an autoimmune disease, a neurodegenerative disease, an inflammatory disease, a cognitive disorder, and a psychiatric disorder in a subject, the method comprising exposing at least a part of the subject to the LF-MF. Methods for treatment of diseases and disorders in a subject, as well as uses of a device for such treatments, are not a part of the invention.

### Characteristics of the modulated low frequency magnetic field

In any of the systems, devices, uses and methods described herein, the carrier frequency may be from 360 to 450 Hz, for example from 365 to 450 Hz, from 370 to 445 Hz, from 390 to 440 Hz, or from 427 to 438 Hz. Preferably the carrier frequency is 432 Hz.

In any of the systems, devices, uses and methods described herein, the modulation frequency may be from 0.5 to 100 Hz, for example the modulation frequency is less than 50 Hz, or less than 30 Hz. Suitably the modulation frequency is from 0.5 to 30 Hz, from 0.5 to less than 30 Hz, from 3 to 30 Hz, from 3 to less than 30 Hz, from 3 to 28 Hz, from 6.5 to 11.5 Hz or from 7 to 10.5 Hz. Preferably the modulation frequency corresponds to a Schumann resonance mode. Accordingly the modulation frequency may be within the range of 7.50 to 8.00 Hz, such as 7.83 Hz; or within the range of 14.0 to 14.5 Hz, such as 14.1 Hz; or within the range of 20.0 to 21.0 Hz, such as 20.3 Hz; or within the range of 26.0 to 27.5 Hz, such as 26.4 Hz. Thus it may be that the modulation frequency is selected from: about 7.83 Hz, about 14.1 Hz, about 20.3 Hz and about 26.4 Hz. Most preferably the modulation frequency is 7.83 Hz.

In any of the systems, devices, uses and methods described herein, MF has a field strength of from 0.5 to 250 µT. Thus it may be that the MF is less than 230 µT, for example 200 µT or less, 100µT or less, 50 µT or less, 30 µT or less, 20 µT or less 15 µT or less or 11 µT or less. Suitably the MF is from 1 to 250 µT, from 5 to 250 µT, from 5 to 220 µT, from 5 to 200 µT, from 10 to 200 µT, from 15 to 200 µT, from 25 to 200 µT, from 25 to 200 µT, from 20 to 120 µT, from 20 to 100 µT, from 20 to 50 µT, from 20 to 40 µT, from 0.5 to 50 µT, from 0.5 to 40 µT, from 0.5 to 30 µT, from 0.5 to 20 µT, from 0.5 to 15 µT, from 0.5 to 11 µT, or 0.5 to 12 µT For example the MF field strength may be about 200 µT, about 100 µT, about 30 µT, or about 11 µT.

In any of the systems, devices, uses and methods described herein, the carrier frequency is preferably 432 Hz. The maximum MF strength is preferably 200 µT. For example the MF preferably varies according to an amplitude-modulated signal with a carrier frequency within the range of 400 to 450 Hz and a modulation frequency within the range of 3 to 30 Hz, such as a modulation frequency corresponding to a Schumann resonance mode, for example within the range of 7.50 to 8.00 Hz, such as 7.83 Hz; or within the range of 14.0 to 14.5 Hz, such as 14.1 Hz; or within the range of 20.0 to 21.0 Hz, such as 20.3 Hz; or within the range of 26.0 to 27.5 Hz, such as 26.4 Hz. The modulation frequency is preferably within the range of 3 to 30 Hz, such as from 3 to less than 30 Hz, e.g. within the range of 7.0 to 28.0, more preferably 7.83 Hz. The amplitude modulation may be of any waveform type, such as sine, square, saw tooth, or triangular. However, the modulation frequency is preferably a sinusoidal modulation frequency, more preferably a sinusoidal modulation frequency within the range of 3 to 30 Hz, such as 7.83 Hz.

In certain embodiments of any of the systems, devices, uses and methods described herein the carrier frequency is from 427 to 438 Hz; the modulation frequency is from 7.50 to 8.00 Hz (suitably wherein the modulation frequency has a sinusoidal waveform); and the magnetic field strength is from 0.5 to 250 µT (e.g. wherein the maximum magnetic field is about 200 µT, about 100 µT, about 30 µT, or about 11 µT). Thus it may be that the magnetic field is from 1 to about 200 µT, 1 to about 100 µT, about 1 to 30 µT, or 1 to about 11 µT. In certain embodiments of any of the systems, devices, uses and methods described herein the carrier frequency is about 432 Hz; the modulation frequency is about 7.83 Hz (suitably wherein the modulation frequency has a sinusoidal waveform); and the magnetic field strength is from 0.5 to 250 µT (e.g. wherein the maximum magnetic field is about 200 µT, about 100 µT, about 30 µT, or about 11 µT). Thus it may be that the magnetic field is from 1 to about 200 µT, 1 to about 100 µT, about 1 to 30 µT, or 1 to about 11 µT.

In preferred embodiments of any of the systems, devices, uses and methods described herein the carrier frequency is 432 Hz and the modulation frequency is about 7.83 Hz. Suitably in these embodiment the maximum MF field strength is 200 µT, for example from 0.5 to 200 µT, from 0.5 to 100 µT, from 0.5 to 50 µT, from 0.5 to 30 µT, from 0.5 to 12 µT, or from 0.5 to 11 µT, for example about 100 µT, about 30 µT, or about 11 µT.

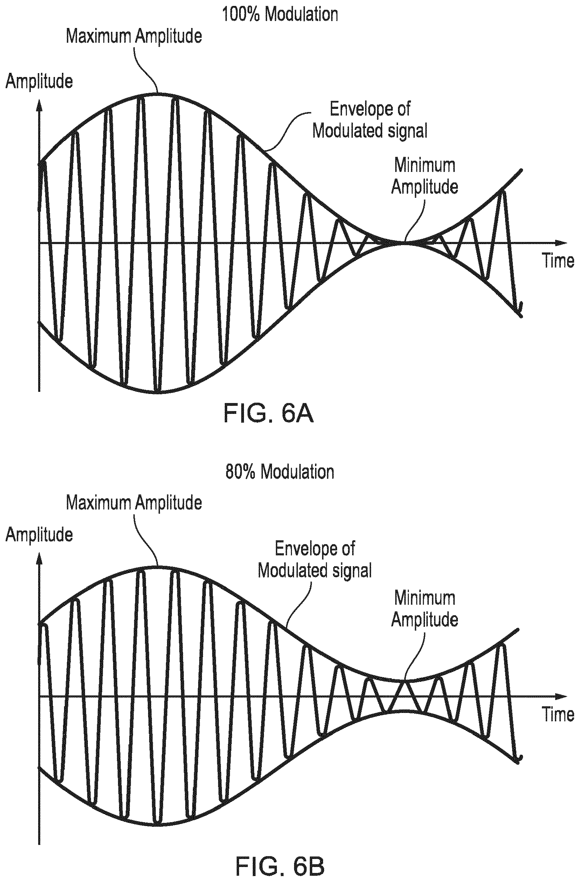

In embodiments of any of the systems, devices, uses and methods described herein the modulated LF-MF may be applied in continuous form or in a pulsed form. When the modulated LF-MF is applied in a pulsed form there are distinct periods where the carrier signal and/or the modulation signal is absent resulting in packets or pulses of the modulated LF-MF are applied. For example, the modulated LF-MF may be applied as pulses followed by a period when there is no applied carrier and modulation signal. When the modulated LF- MF is applied continuously both the carrier signal and the modulation signal are applied continuously (i.e. with no distinct "off" periods of the carrier and/or the modulation signal). By way of an example, when the carrier frequency is modulated using a sine wave modulation, the carrier frequency is sinusoidally modulated at the modulation frequency throughout the exposure without any distinct breaks in the applied modulated LF-MF. Figures 6A and 6B illustrate such a continuous modulated LF-MF. In preferred embodiments the modulated LF-MF is a continuous modulated LF-MF.

In any of the systems, devices, uses and methods described herein the subject, organic cells or cellular tissue may be exposed to the modulated LF-MF for a time suitable to provide the desired effect. The specific duration and frequency of exposure will depend on the particular situation and the desired effect of the modulated LF-MF. For example, where the system is used for cosmetic or non-therapeutic application the subject or sample may be exposed to the LF-MF for 5 minutes to 4 hours, for example from 5 minutes to 2 hours, or from 10 minutes to 1 hour. When the system is used for therapeutic applications the subject, or part of the subject, may be exposed to the LF-MF substantially continuously (e.g. during wound healing or to promote tissue regeneration). Thus in therapeutic applications the subject, or an area on the subject may be exposed to the LF-MF for 1 hour or more, 2 hours or more, 4 hours or more, 8 hours or more, 12 hours or more, 24 hours or more, 1 week or more, 1 month or more, 3 months or more. For example from 1 hour 3 months or more, from 1 day to 3 months, from 1 week to 3 months, from 1 week to 2 months or from 1 week to 1 month. When the system is used to enhance proliferation of stem or progenitor cells ex-vivo, for example in a cell culture vessel, the cells are suitable exposed to the LF-MF substantially continuously throughout the process to maximise proliferation of the cells ex *vivo.* However, shorter exposure times are also envisaged wherein the cells are exposed to the LF-MF for only part of the ex *vivo* process, for example for 10 minutes to 3 months, 12 hours to 1 month, 24 hours to 1 week, 1 day to 3 months, 1 week to 12 weeks or 1 week to 10 weeks.

### BRIEF DESCRIPTION OF THE DRAWINGS

Embodiments of the invention are further described hereinafter with reference to the accompanying drawings, in which:
Figures 1A-C schematically show embodiments of the MF exposure system as disclosed herein in the form of a chair.
Figures 2 schematically shows another embodiment of the MF exposure system as disclosed herein in the form of a room.
Figure 3 is a schematic diagram showing a circuit used to produce MFs in an embodiment of the MF exposure system as disclosed herein.
Figures 4 schematically shows a further embodiment of the MF exposure system as disclosed herein in the form of an egg-shaped chair.
Figure 5 is a diagram presenting the three main maturation phases involved in epidermal homeostasis *in vivo* and *in vitro.*
Figure 6 illustrates an amplitude-modulated carrier frequency (driver frequency), such as 432/7.83 Hz described herein. 6A illustrates 100% amplitude modulation and 6B approximately 80% amplitude modulation.
Figures 7A and 7B show extrapolated cell numbers on a 10⁶ scale of primary human stem and progenitor keratinocytes from young (Figure 7A) and adult (Figure 7B) donors grown in an electromagnetic coil with no MF generated (coil Off) or with a continuous 432/7.83 Hz MF at 200 µT. Cells were continuously exposed while expanded to 80% confluency over 6 passages during the proliferative phase 1 of epidermal homeostasis (see Figure 5). The subgraphs show the zoomed scale for the respective passages 0 to 1, 1 to 2, 2 to 3 and 4 to 5.

Figures 8A and 8B shows relative cell numbers of primary human stem and progenitor keratinocytes from young (Figure 8A) and adult (Figure 8B) donors continuously exposed to no MF (coil Off), or a 7.83 Hz or 432/7.83 Hz MF at 200 µT while expanded to 80% confluency over 1 passage (i.e. Phase 1 in Figure 5).

Figure 9 shows the relative cumulative cell counts of primary human stem and progenitor keratinocytes from young donors continuously exposed to no MF (coil Off), 432 Hz or 432/7.83 Hz MF while expanding 36,000 cells per passage to 80% confluency over 6 passages (Phase 1, Figure 5). The trace marked ** are the cells exposed to 432 Hz. The trace marked *** are the cells exposed to 432/7.83 Hz MF.

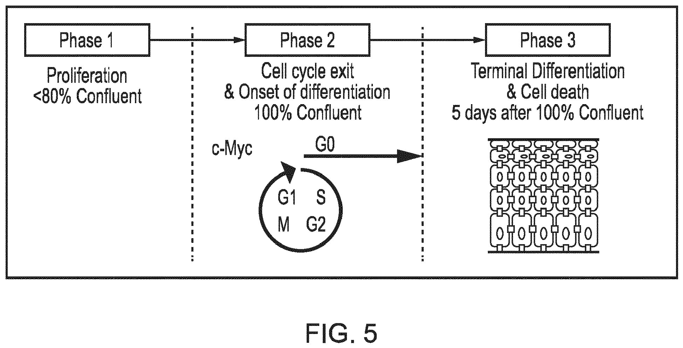

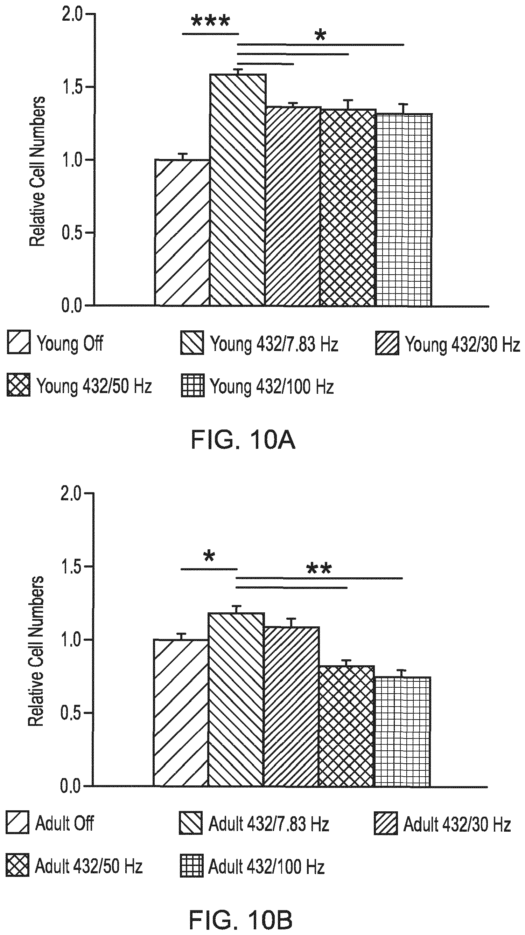

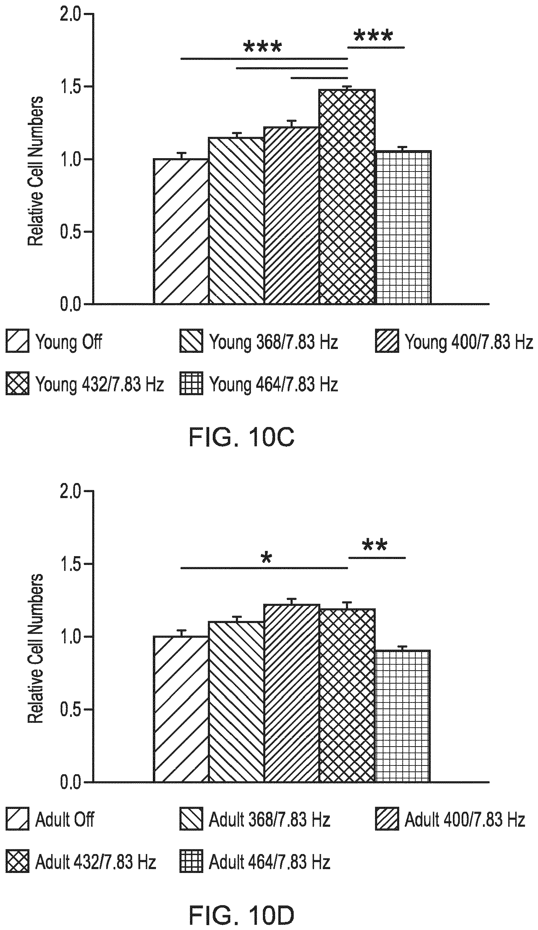

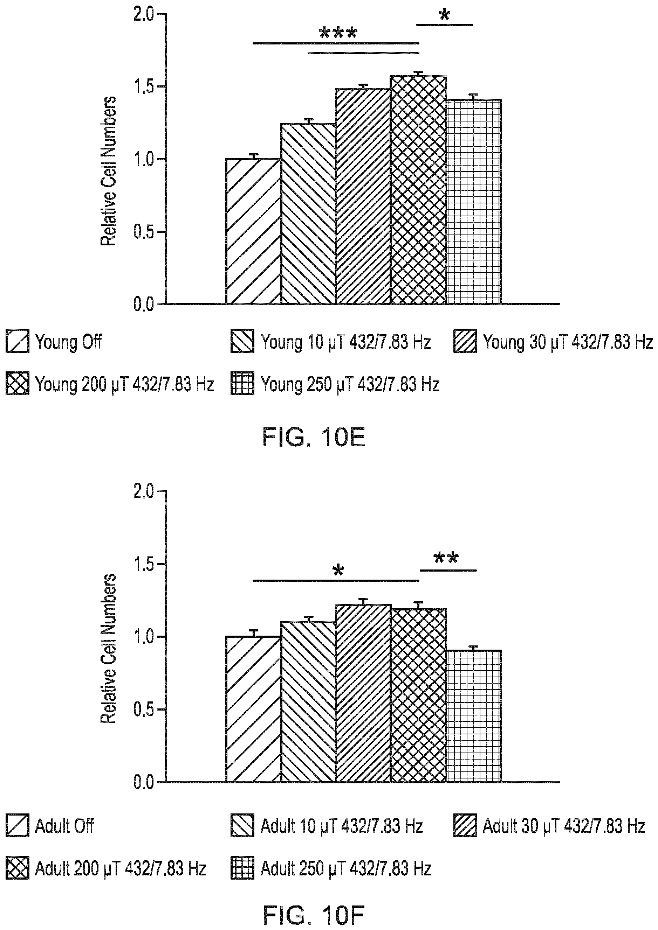

Figures 10A, 10B, 10C, 10D, 10E and 10F compare the effect of a range of (10A, 10B) modulation frequencies, (10C, 10D) driver frequencies and (10E, 10F) magnetic field intensities on proliferation (Phase 1, Figure 5) of continuously exposed primary young and adult stem and progenitor cells, respectively. Figures 10A and 10B show cells exposed to no MF (coil Off), 432/7.83 Hz, 432/30 Hz, 432/50 Hz and 432/100 Hz. Figures 10C and 10D show the effects of a range of diver frequencies on cells exposed to no MF (coil Off), 368/7.83 Hz, 400/7.83 Hz, 432/7.83 Hz and 464/7.83 Hz. Figures 10E and 10F show the effects of magnetic field intensities on cells exposed to no MF (coil Off), or 368/7.83 Hz at 10 µT, 30 µT, 200 µT or 250 µT.

Figures 11A and 11B show relative dead cell numbers of primary human stem and progenitor keratinocytes from young (Figure 11A) and adult (Figure 11B) donors exposed to no MF (coil Off) or continuous 7.83 Hz or 432/7.83 Hz MF during expansion to 80% confluency for 1 passage (Phase 1, Figure 5).

Figures 12A and 12B show contact inhibition and metabolic turnover of primary human stem and progenitor keratinocytes from young (Figure 12A) and adult (Figure 12B) donors continuously exposed to no MF (coil Off), 7.83 Hz or 432/7.83 Hz MF during all 3 metabolic phases (see Figure 5) from 1 to 4 passages.

Figure 13 shows the relative shortening of telomere length (read-out for aging) (mean ratio of ddCt values) over 6 passages in primary human stem and progenitor keratinocytes from young donors exposed to no MF (coil Off) or 432/7.83 Hz MF.

Figures 14A and 14B show relative protein levels of heat shock protein 47 (Hsp47) (Figure 14A) and Hsp90 (Figure 14B) normalised to tubulin of primary human stem and progenitor keratinocytes from young donors exposed to no MF (coil Off) or 432/7.83 Hz MF in fully supplemented medium or starvation medium comprised of 25% medium/75%PBS, respectively.

Figure 15 shows an embodiment of the MF exposure system as disclosed herein in the form of a chair used in the EEG pilot study described in the Examples.

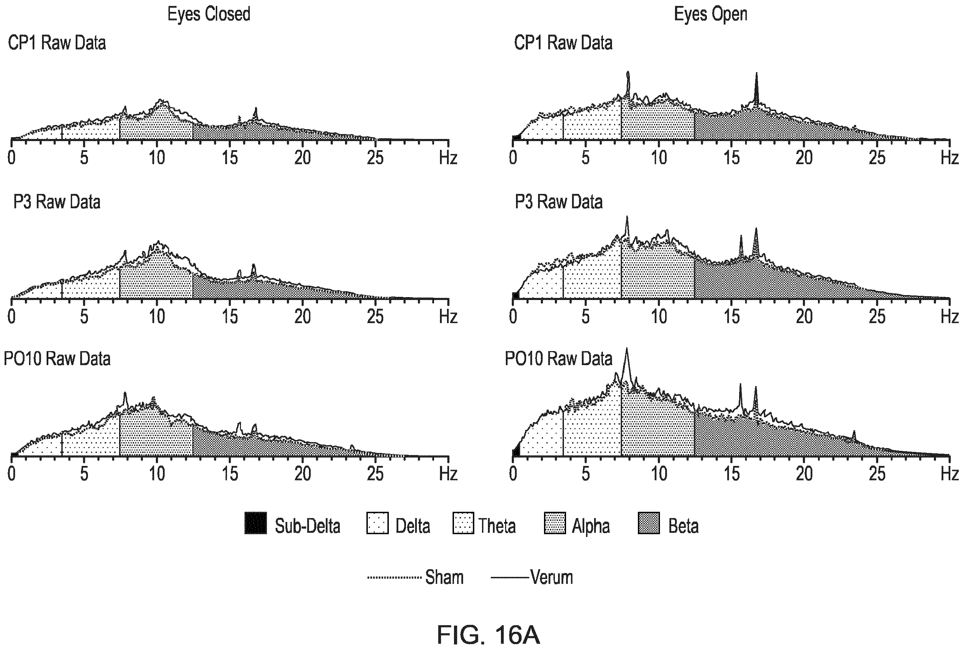

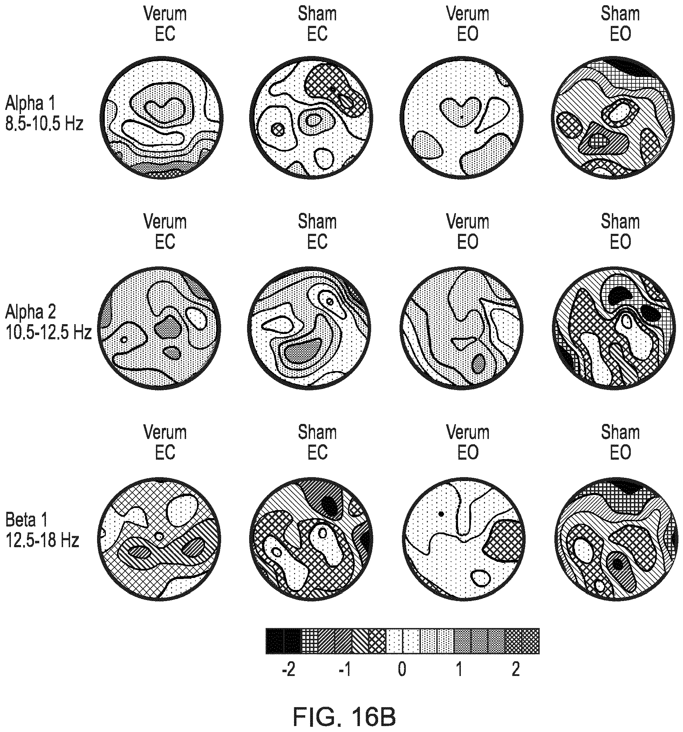

Figures 16A and 16B show results from a blind cross-over pilot study of EEGs measured on 4 individuals over 18 sessions showing differences in mainly alpha bands in the parental occipital scalp with eyes closed (EC) and eyes open (EO) under verum (432/7.83 Hz at 30 µT) compared to sham (no MF) conditions. (Figure 16A) Graphical display of the average power of each frequency band for sham and overlaid verum (solid curve) conditions, and (Figure 16B) t-maps of changes in lower and higher alpha bands as well as the beta1 band over the scalp (prefrontal cortex towards top of the page; parietal occipital cortex towards bottom page). Levels above 1 and below -1 point towards significant changes calculated by the MATLAB add-on "Randomization Graphical User interface" (Ragu (Habermann, M., Weusmann, D., Stein, M. & Koenig, T. A Student's Guide to Randomization Statistics for Multichannel Event-Related Potentials Using Ragu. Front Neurosci 12, 355, doi:10.3389/fnins.2018.00355 (2018))

### DETAILED DESCRIPTION

As used herein, the term "subject" refers to a human or an animal (e.g. non-human primates; domestic and farm animals, such as dogs, horses, cats, cattle sheep, mice, rats, or rabbits). Suitably the subject is a human.

As used herein, the term "a healthy subject" means a subject (e.g. a human) absent of any clinical signs of physical diseases and infections.
The term "stem cell" is used herein to refer to a cell that has the ability both to self-renew, and to generate differentiated progeny (see Morrison et al. (1997) Cell 88:287-298). Generally, stem cells also have one or more of the following properties: an ability to undergo asynchronous, or asymmetric replication; i.e., where the two daughter cells after division can have different phenotypes; extensive self-renewal capacity; capacity for existence in a mitotically quiescent form; and clonal regeneration of all the tissue in which they exist, for example the ability of hematopoietic stem cells to reconstitute all hematopoietic lineages. "Progenitor cells" differ from stem cells in that they typically do not have the extensive self-renewal capacity, and often can only regenerate a subset of the lineages in the tissue from which they derive. In certain embodiments the stem cells are multipotent stem cells, which include adult stem cells that are capable of differentiating into specific cell types, examples include hematopoietic stem cells, mesenchymal stem cells and neural stem cells. In certain embodiments the stem cells are pluripotent stem cells, cells which can differentiate into any type of cell including embryonic stem cells, perinatal stem cells (umbilical cord stem cells) and induced pluripotent stem cells (iPSCs). In certain embodiments the stem cell is a multipotent stem cell or an induced pluripotent stem cell. In certain embodiments the stem cell is a not an embryonic stem cell.

The stem cells or progenitor cells may be mammalian stem or progenitor cells, where the term "mammalian" refers to any animal classified as a mammal, including humans; non-human primates; domestic and farm animals (e.g. dogs, cats, horses, cattle, sheep, mice, rats or rabbits). Suitably the stem cell is a human stem cell.

Stem cells include adult stem cells. Adult stem cells are also referred to as somatic or tissue stem cells. Adult stem cells are resident in differentiated tissue, but retain the properties of self-renewal and ability to give rise to multiple cell types, usually cell types typical of the tissue in which the stem cells are found. Numerous examples of somatic stem cells are known to those of skill in the art, including muscle stem cells, hematopoietic stem cells, epithelial stem cells, neural stem cells, mesenchymal stem cells, mammary stem cells, intestinal stem cells, mesodermal stem cells, endothelial stem cells, olfactory stem cells, dental pulp stem cells or neural crest stem cells.

In some embodiments, the stem cell is a hematopoietic stem cell (HSC). HSCs are mesoderm-derived cells that can be isolated from bone marrow, blood, cord blood, foetal liver and yolk sac. HSCs can repopulate the erythroid, neutrophil-macrophage, megakaryocyte and lymphoid hematopoietic cell lineages in vivo. In vitro, HSCs can be induced to undergo at least some self-renewing cell divisions and can be induced to differentiate to the same lineages as is seen in vivo. As such, HSCs can be induced to differentiate into one or more of erythroid cells, megakaryocytes, neutrophils, macrophages, and lymphoid cells.

In other embodiments, the stem cell is a neural stem cell (NSC). Neural stem cells (NSCs) are capable of differentiating into neurons, and glia (including oligodendrocytes, and astrocytes). A neural stem cell is a multipotent stem cell which is capable of multiple divisions, and under specific conditions can produce daughter cells which are neural stem cells, or neural progenitor cells that can be neuroblasts or glioblasts, e.g., cells committed to become one or more types of neurons and glial cells respectively.

In other embodiments, the stem cell is a mesenchymal stem cell (MSC). MSCs originally derived from the embryonal mesoderm and isolated from adult bone marrow, can differentiate to form muscle, bone, cartilage, fat, marrow stroma, and tendon.

In a preferred embodiment the stem cell is a keratinocyte stem cell.

In certain embodiment the stem cells of are aged adult stem cells. For example, an aged adult stem cell is an adult stem cell obtained from, or present in, a human individual greater than 30 years, greater than 40 years, greater than 50 years, greater than 60 years, or greater than 70 years. For example, an aged adult stem cell obtained from, or present in, a human individual who is aged from 30 years to 70 years.

In certain embodiments the stem cells are young adult stem cells, for example adult stem cells obtained from, or present in, a human individual less than 10 years old, or less than 6 years old for example aged from 0 month to 10 years, or from 1 month to 6 years.

Reference to "primary cells" refers to cells that have been obtained from a subject. Examples of primary cells include epithelial cells, endothelial cells, fibroblasts, melanocytes, keratinocytes, neurons, astrocytes, hepatocytes, skeletal muscle cells, smooth muscle cells, osteoblasts, myocytes, chondrocytes, adipocytes, synoviocytes, hair cells, blood cells stem cells or progenitor cells. Primary cells may be obtained using well known methods, for example via a tissue biopsy followed separation/isolation of the primary cells of interest. Cell separation and isolation methods are well known and include immunomagnetic cell separation, fluorescent-activated cell sorting, centrifugation methods (e.g. density gradient centrifugation, immunodensity cell separation, cell sedimentation, cell adhesion methods or microfluidic cell separation).

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse effect attributable to the disease. "Treatment," as used herein, covers any treatment of a disease in a mammal, e.g., in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

Methods of treatment performed on a human or animal body as well as uses of a device for such treatments are not a part of the invention.

Reference to a condition being "associated with" stem or progenitor cells refers to the condition being caused directly or indirectly by stem or progenitor cells, for example wherein the disease or condition results from, or is mitigated by, reduced stem cell proliferation, stem cell or progenitor cell senescence, stem cell or progenitor cell stress, stem cell or progenitor cell age, and/or reduced stem cell or progenitor cell differentiation.

Unless stated otherwise, references herein to "magnetic field strength", "MF strength", "magnetic field intensity" or "field strength intensity" refer to the root mean square (rms) of the magnetic flux density (B) produced by the system described herein and is a vector quantity that determines the force on a moving charge or charges (electric current). Magnetic flux density is expressed in tesla (T).

The term "about" herein refers to +/- 10% of the figure quoted.

The invention is defined in the following claims. Other embodiments, examples, items and, in particular, therapeutic methods, are not a part of the invention.

Throughout the description and claims of this specification, the words "comprise" and "contain" and variations of them mean "including but not limited to", and they are not intended to (and do not) exclude other components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

### Magnetic Field Exposure System

Disclosed herein is a MF exposure system for use in exposing organic cells, cellular tissue or at least a part of a subject to a LF-MF. For example, the system may be a ring or cylinder configured to surround, for example, an arm or leg of the subject to be exposed, or a chair, a room or a chamber.

The system comprises a magnetic field generator comprising one or more coils configured to produce a magnetic field that varies according to an amplitude modulated signal with a carrier frequency within the range of 360 to 450 Hz (e.g. 400 to 450 Hz), and wherein the magnetic field has a maximum magnetic field strength within the range of 0.5 to 250 µT (e.g. 10 to 200 µT). The magnetic field generator may comprise an electric generator and an amplifier feeding one or more coils configured to produce a MF that varies according to an amplitude-modulated signal with a carrier frequency within the range of 360 to 450 Hz (e.g. 400 to 450 Hz) and a MF strength within the range of 0.5 to 250 µT (e.g. 0.5 to 200 µT, or 10 to 200 µT). The amplitude modulated magnetic field has a modulation frequency of from 0.5 to 100 Hz, for example from 3 to less than 30 Hz, or from 3 to 28 Hz.

In a preferred embodiment, the system comprises a magnetic field generator comprising an electric generator and an amplifier feeding one or more coils configured to produce a MF that varies according to an amplitude-modulated signal with a carrier frequency within the range of 400 to 450 Hz, a modulation frequency corresponding to a Schumann resonance mode (preferably 7.83 Hz), and a MF strength within the range of 0.5 to 250 µT (e.g. 0.5 to 200 µT, or 10 to 200 µT).

The one or more coils of the MF exposure system may be configured to at least partially surround the subject to be exposed to said LF-MF.

The one or more coils of the MF exposure system may be one or more copper coils.

The size, shape and type of coil system can be decided according to the biological system to be exposed. Circular coil systems, such as Helmholtz coil, may be used for generating MFs over a small volume whereas square coil systems such as Merritt and Ruben coils, may generate MF over a large volume, for example for whole body exposure or within a room or chamber. For whole body exposure, it is preferred that the generated MF is substantially uniform.

Numerous coil configurations and geometries may be used to generate the modulated LF-MF. The specific coil geometry used will be determined by the desired application and volume of LF-MF required. The design and optimisation of the coil geometries and orientations may be carried out using known coil configurations and methods (e.g. as described in Kirschvink JL Uniform magnetic fields and double-wrapped coil systems: improved techniques for the design of bioelectromagnetic experiments. Bioelectromagnetics 13:401-411). In certain embodiments the coil is a Helmholtz coil, wherein two or more circular coils of substantially the same diameter are coaxially spaced apart. In other embodiments Merritt coils may be used wherein square coils are arranged to provide a uniform magnetic field within the coils (Merritt et. al., (1983) Uniform magnetic-field produced by three, four, and five square coils. Rev Sci Instrum 54:879-882). For example Merritt coils may be arranged orthogonally with e.g. 2 to 5 (or more) coils per axis spaced apart from one another. This arrangement provides an inner volume defined by the coils in which the modulated LF magnetic field produced by a current passed through the coils is substantially uniform.

In certain embodiments the one or more coils is a Helmholtz coil. The Helmholtz coil comprises two substantially identical circular coils that are co-axially spaced apart, wherein the spacing between the coils is the substantially the same as the radius of the coils. The coils are supplied with the same input electrical current, which results in the generation of a substantially uniform magnetic field within the space between the two coils. The use of a Helmholtz coil in the system enables the subject (or part of the subject), or the sample (e.g. cells) to be placed in the space between the coils such that the subject or sample is exposed to a substantially uniform MF.

The maximum MF strength of the MF generated by the MF generator of the MF exposure system may be set within the acceptable limits according to national recommended exposure levels. For example, as set out in the International Commission on Non-Ionizing Radiation Protection's (ICNIRP's) guidelines 1998 and 2010. Suitably the MF strength of the LF-MF is within the range of 0.5 to 200 µT when the system is used to expose a human subject to the modulated LF-MF such as within the range of 1 to 200 µT, 5 to 200 µT, 10 to 200 µT, 25 to 200 µT, 50 to 200 µT, 100 to 200 µT, 150 to 200 µT, 1 to 40 µT, 1 to 20 µT, 1 to 15 µT, or 1 to 11 µT. In certain embodiments, the maximum magnetic field strength of the LF-MF is 200 µT. In other embodiments the maximum magnetic field strength is lower, for example a maximum magnetic field of about 100 µT, about 30 µT, about 30 µT, about 20 µT, about 15 µT, about 12 µT, or about 11 µT. Where the system is used to, for example expose cells to the modulated LF-MF *ex-vivo* (e.g. to enhance cell proliferation *in vitro*), a higher maximum magnetic field strength may be used, for example any of the magnetic field strengths disclosed herein up to 250 µT. The maximum magnetic field refers to the root mean square (rms) value of the magnetic field strength. The magnetic field strength may be determined using known methods, for example using a suitable Gauss meter.

Reference herein to the modulated LF-MF, refers to the modulated magnetic field produced by the coil(s) when a temporally-varying current is passed through the coil(s). Thus references to the magnetic field-strength herein refer to the magnetic field produced by electrical current passing through the coil(s) of the systems described herein. As will be appreciated by the skilled person other electrical and magnetic fields may also be present as a result of for example the earth's magnetic field and background fields field from electric equipment. In certain embodiments the effects of external electrical and/or magnetic background may be reduced or eliminated by, for example, locating the system in a Faraday cage and/or use of µ-metal layers surrounding the system to screen out unwanted external electrical and/or magnetic background.

The MF exposure system as disclosed herein may be configured to expose at least 50%, such as 50-75%, of the subject's body surface to said MF. For example, the MF exposure system may be configured to expose the upper body, including head and torso, of the subject to said MF. In some embodiments, the MF exposure system may be configured for whole body exposure of the subject. In such embodiments, the MF exposure system provides cranial as well as peripheral MF exposure of the subject.

Alternatively, the MF exposure system as disclosed herein may be configured to provide only cranial MF exposure of the subject. In such embodiments, only the brain region of the subject is exposed to said MF.

Still alternatively, the MF exposure system as disclosed herein may be configured to provide only peripheral (transcutaneous) MF exposure of the subject. In such embodiments, the brain of the subject is not exposed to said MF.

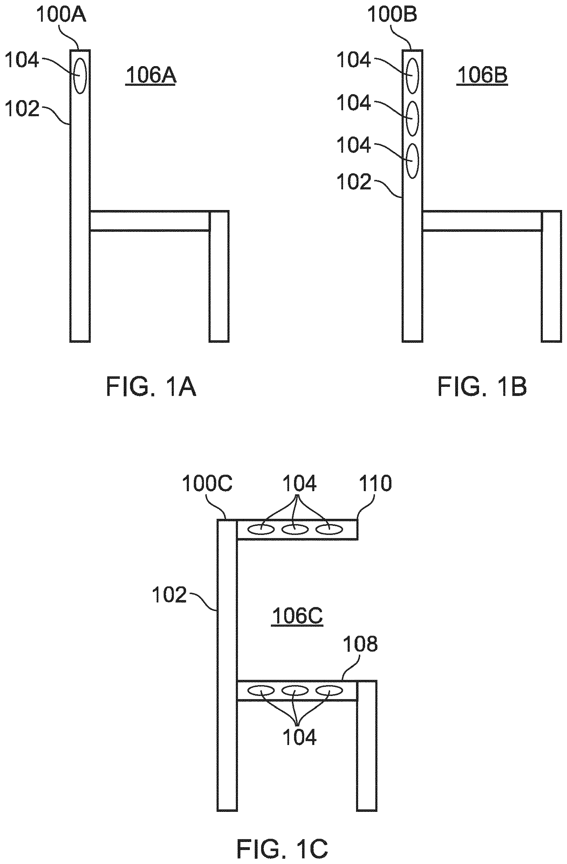

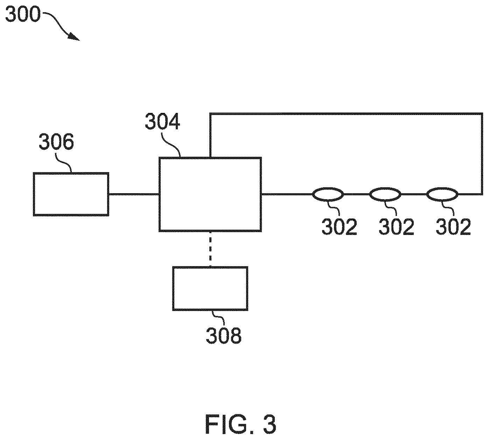

Figure 1A shows a MF exposure system in an embodiment of the present invention. In the illustrated embodiment the MF exposure system comprises a chair 100A having a backrest 102 into which a coil 104 is integrated. The coil 104 is electrically connected to a driver circuit 300 comprising an electric generator and amplifier as shown in Figure 3, which driver circuit is configured to cause a temporally-varying current to pass through the coil 104.

As will be well understood by the skilled person, when a current passes through the coil 104, a MF having an intensity proportional to the magnitude of the current is generated within and around the coil. Passage of a temporally-varying current through the coil therefore generates a temporally-varying MF.

In particular, the coil 104 is arranged such that, when a current is passes through the coil 104, a MF is generated in and around region 106A, in which at least a portion of a subject's head is likely to be located when the subject is sat on the chair 100.

Figure 1B shows a chair 100B in a similar embodiment to that shown in Figure 1A. However, in Figure 1B three separate coils 104 are provided within the backrest 102. In this way, the region 106B in which a MF is generated is expanded, and may for example include the region in which a subject's head and torso are likely to be located when the subject is sat on the chair 100B.

Figure 1C shows a chair 100C in a further embodiment of the present invention, in which the coils 104 are located in a seat portion 108 and a top portion 110 of the chair 100C. In this way a substantially uniform MF may be generated in region 106C, which is likely to contain the entire head and torso of a subject sitting in the chair 100C.

Particularly, at least parts (such as head and/or torso) of a subject sitting in any of chairs 100A, 100B or 100C are exposed to the MF that varies according to an amplitude-modulated signal with a carrier frequency within the range as defined herein, for example, 400 to 450 Hz (e.g. 432 Hz), a modulation frequency corresponding to a Schumann resonance mode (e.g. 7.83 Hz), and a MF strength within the range of 10 to 200 µT. More particularly, at least parts (such as head and/or torso) of a subject sitting in any of chairs 100A, 100B or 100C are exposed to a homogenous MF that varies according to an amplitude-modulated signal with a carrier frequency within the range of 400 to 450 Hz (e.g. 432 Hz), a modulation frequency corresponding to a Schumann resonance mode (e.g. 7.83 Hz), and a MF strength within the range of 0.5 to 250 µT (e.g. 10 to 200 µT).

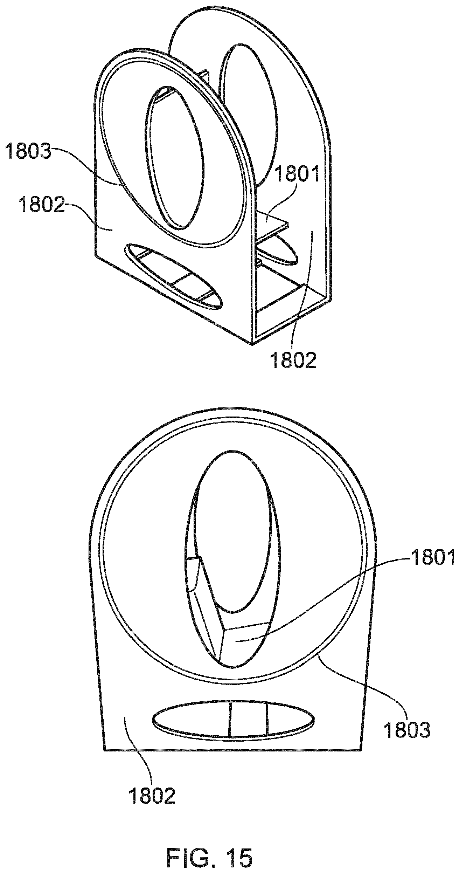

Although the chair 100C shown in Figure 1C is shown with a covered top portion 110, it will be understood that in some embodiments side walls may also be provided, such that at least part of a subject can be located within a cavity defined by the backrest 102, the seat portion 108, the top portion 110 and the side walls (not shown). Coils may be located in the side walls and/or backrest as well as, or instead of, the coils located in the seat portion 108 and the top portion 110. The system in Figure 15 shows a seat 1801 located in the cavity formed between two sidewalls 1802. Circular coils 1803 of the same diameter are located in each sidewall 1802, wherein the coils 1803 are located co-axially thereby forming a Helmholtz coil. The coils 1803 are electrically connected to a driver circuit 300 as shown in Figure 3, which driver circuit is configured to cause a temporally-varying current to pass through the coils 1803. When current is passed through the coils 1803, a substantially uniform magnetic field is generated in the space between the two coils 1803. In use, when a subject sits on the chair 1801, the subject will be exposed to the modulated LF-MF generated between the coils 1803. The size of the coils can be configured to expose just part of the body, for example the head or the torso. Alternatively, larger coils may be used to expose substantially all of the subject to the modulated LF-MF.

Furthermore, in some embodiments a cavity in which at least part of a subject can be located could be defined by a single curved wall having one or more coils located therein. A chair including such a cavity may be referred to as an egg-pod chair.

In an embodiment, the system is a chair comprising a hollow egg-shaped body forming a seat and a backrest, and one or more coils are integrated in the hollow egg-shaped body of the chair to generate the LF-MF.

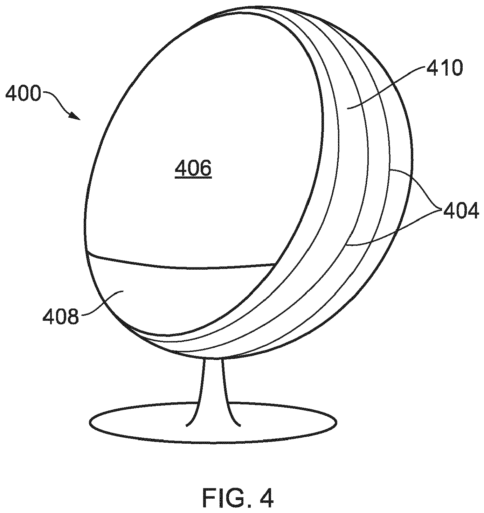

Figure 4 shows a chair 400 in a further embodiment of the present invention. The chair 400 comprises a hollow egg-shaped body forming a seat 408 and a backrest 410, in which a plurality of coils 404 are integrated. In this way a substantially uniform MF will be generated in region 406, which is to contain the entire head and torso of a subject sitting in the chair 400. The chair in Figure 4 may include a plurality of copper coils 404. The plurality of coils 404 is electrically connected to a driver circuit 300 as shown in Figure 3, which driver circuit is configured to cause a temporally-varying current to pass through the coils 404.

In some embodiments, the parts of chair that define a cavity in which a user may sit (e.g. an egg-pod chair as shown in Figure 4, a chair 100C as shown in Figure 1C or the chair shown in Figure 15) may be provided with an outer lining that shields the user from ambient EMFs, thereby ensuring that the user only experiences the well-defined stimulation produced by the chair 100C or 400 whilst using the chair 100C or 400. Such a lining may be formed for example of a mu-metal (µ-metal), which is a nickel-iron soft ferromagnetic alloy that is used for shielding sensitive electronic equipment against static or low-frequency MFs. An exemplary composition comprises 77% nickel, 16% iron, 5% copper, and 2% chromium or molybdenum.

Thus, in a further embodiment, the system is a chair comprising a hollow egg-shaped body forming a seat and a backrest, one or more coils are integrated in the hollow egg-shaped body of the chair to generate the LF-MF, and the hollow egg-shaped body of the chair is electromagnetically shielded.

In a still further embodiment, the system is a chair comprising a hollow egg-shaped body forming a seat and a backrest, one or more coils are integrated in the hollow egg-shaped body of the chair to generate the LF-MF, and the hollow egg-shaped body comprises a lining formed of a mu-metal.

In another embodiment, the system is a chair comprising a hollow egg-shaped body forming a seat and a backrest, wherein at least two orthogonal copper coils are integrated in the hollow egg-shaped body of the chair to generate the LF-MF. The hollow egg-shaped body of the chair may be electromagnetically shielded, particularly the egg-shaped body may comprise a lining formed of a mu-metal.

In a still further embodiment, the system is a chair comprising a hollow egg-shaped body forming a seat and a backrest, wherein a first plurality of copper coils (e.g. 2-5 copper coils) and a second plurality of copper coils (e.g. 2-5 copper coils) are integrated in the hollow egg-shaped body of the chair, said first plurality of copper coils extending orthogonal to said second plurality of copper coils. The hollow egg-shaped body of the chair may be electromagnetically shielded, particularly the egg-shaped body may comprise a lining formed of a mu-metal.

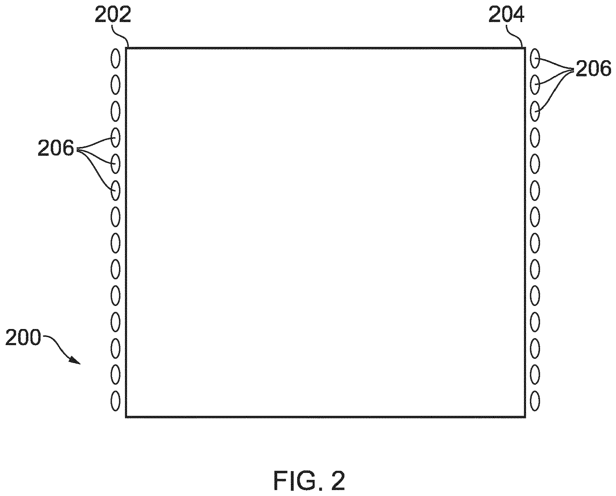

Figure 2 shows a room (or chamber) 200 of a building in a further embodiment of the present invention. The side walls 202, 204 of the room 200 each contain a plurality of coils 206, such that when a temporally-varying current is passed through the coils 206, a corresponding temporally-varying MF is generated within the room 200. When a temporally-varying current is simultaneously passed through all of the coils 206, a corresponding temporally-varying MF may substantially fill the room 200.

The coils may be arranged such that the direction of the MF passing through the coils at a given point in time is in the same direction (i.e. vertically up or down) for all of the coils, thereby reducing spatial variations in the MF. A subject present in the room or chamber 200 is exposed to a MF that varies according to an amplitude modulated signal with a carrier frequency within the range described herein (e.g. 400 to 450 Hz (e.g. 432 Hz)) and a MF strength within the range of 0.5 to 250 µT (e.g.10 to 200 µT). Particularly, a subject present in the room or chamber 200 is exposed to a MF that varies according to an amplitude-modulated signal with a carrier frequency within the range of 400 to 450 Hz (e.g. 432 Hz), a modulation frequency corresponding to a Schumann resonance mode (e.g. 7.83 Hz), and a MF strength within the range of 10 to 200 µT. More particularly, a subject present in the room or chamber 200 may be exposed to a homogenous MF that varies according to an amplitude-modulated signal with a carrier frequency within the range of 400 to 450 Hz (e.g. 432 Hz), a modulation frequency corresponding to a Schumann resonance mode (e.g. 7.83 Hz), and MF strength within the range of 0.5 to 250 µT (e.g.10 to 200 µT).

The walls, floor and ceiling of the room 200 may all be provided with an outer shielding layer, to prevent interference from ambient EMFs originating outside the room. Again, the shielding layer may be formed of a mu-metal (µ-metal).

Although the embodiments shown in Figures 1 and 2 show one or more coils distributed around the target region in which a subject is to be located to be exposed to the MF, it will be understood that other configurations are also possible. For example, in some embodiments a single large coil may surround the target region, such that all or part of the subject can be located within the coil. This may be particularly useful in the event that the MF exposure system comprises a room in which a subject may be exposed to a MF, or a chair that at least partially surrounds the subject (for example an egg-pod chair).

In further embodiments the system comprises a horizontal platform (e.g. a bed) and one of more coils that surround the platform such that when a temporally-varying current is passed through the coils a corresponding temporally-varying MF is generated around the horizontal platform. In use when a subject lies on the bed, at least part of the subject is exposed to the modulated LF-MF. The system may be configured such that a plurality of coils surrounds the horizontal platform. For example a plurality of coils, (e.g. circular coils) surrounding the horizontal platform may be coaxially located along the horizontal axis of the platform, thereby exposing at least part, or preferably the whole body of a subject lying on the platform to the LF-MF. In further embodiments the system comprises a horizontal platform (e.g. a bed) and one of more coils located above and/or below the platform.

For exposing smaller areas of the body the system may comprise a housing with one of more coils located within the housing such that when a temporally-varying current is passed through the coils a corresponding temporally-varying MF is generated. The housing can then be positioned to expose a specific body part or tissue of a subject to the LF-MF. It may be that the housing is hand held such that the LF-MF can be positioned manually to direct the LF-MF to a target area on the subject. Alternatively, the housing my be, or comprise a frame that can be moved to direct the LF-MF to a particular part of the subject.

In a further embodiment the system is in the form of a cell culture system, said system comprising cell culture vessel defining a cell culture cavity within the vessel and a MF generator with one or more coils configured to produce a MF within the cell culture cavity that varies according to an amplitude-modulated signal with a carrier frequency of from 360 to 450 Hz, wherein the modulation frequency is from 0.5 to 100 Hz; and wherein the MF has a field strength of from 0.5 to 250 µT.

The cell culture cavity is a volume within the cell culture vessel that is suitable for containing cells to be proliferated (cultured). Thereby in use the cells contained within the cell culture cavity are exposed to the modulated LF-MF described herein.

The cell culture vessel may be any suitable vessel for culturing cells such as stem and progenitor cells, for example the vessel may be a petri-dish, a flask, multi-well plate (e.g. a 96-well plate),a bottle, a conical tube, a stacked culture vessel, a 2D tissue culture vessel or a 3D suspension culture vessel, or a larger scale bioreactor vessel. The one or more coils are located in a position such that when a current is applied to the coils, a LF-MF is generated within the cell culture cavity. In use, cells are contained within the cell culture cavity and are exposed to the LF-MF. In certain embodiments a pair of coils may be coaxially spaced apart (e.g. as a Helmholtz coil) and the reaction vessel is located between the coils such that in use the cell culture cavity in the cell culture vessel is exposed to a substantially uniform LF-MF when a current is passed through the coils. In a further embodiment the one or more coils are attached to the walls of the cell culture vessel and are configured to produce a LF-MF in the cell culture cavity. It may be that the one or more coils are embedded in the walls of the cell culture vessel. In a further embodiment the cell culture system further comprises one or more organic cells (e.g. one or more stem or progenitor cells or primary cells) in the cell culture cavity and optionally further comprises a cell culture medium in the cell culture cavity.

Figure 3 shows a schematic representation of a MF generator circuit 300 arranged to produce a temporally-varying MF. The illustrated circuit comprises a plurality of coils 302 arranged in series, and connected to an electric generator 306 and an amplifier 304 controlling the electric output and frequency to the coils. A micro controller 308 may be included for regulating usage. It will be understood that the coils could equally be arranged in parallel or in any other suitable circuit arrangement.

Electric generator 306 is fed via power source 310, and the output frequency is controlled by the amplifier 304. The power source 310 may be a source of DC power, such as a battery, or a source of AC power, such as a mains connection.

Electric generator 306 is operable, under the control of the amplifier 304, to output a predefined, constant, temporally-varying voltage or current signal. In particular, the electric generator 306 and amplifier 304 may be configured to output an amplitude-modulated AC signal, having a carrier frequency as described herein (e.g. 400 to 450 Hz) and a sinusoidal modulation frequency, as illustrated in Figure 6. As will be discussed in more detail below with respect to Example 1, particularly beneficial effects have been observed *in vitro* when cells are exposed to a temporally-varying MF corresponding to the amplitude-modulated signal of 80 to 100% as illustrated in Figures 6A and 6B. In particular, such beneficial effects were observed when the amplitude is modulated by 100% and the modulation signal is sinusoidal with a modulator frequency corresponding to a Schumann resonance mode, such as 7.83 Hz, and the carrier signal has a frequency of 400 to 450Hz, such as 432 Hz.

The first Schumann resonance mode occurs at approximately 7.83 Hz, although any frequency within a range of approximately 7.0 - 10.0 Hz, more particularly 7.5 - 8.0 Hz, may be considered to correspond to the first Schumann resonance within the scope of the present disclosure. Further Schumann resonance frequencies occur at least within the following frequency ranges: 14.0 to 14.5 Hz, more particularly 14.1 Hz; 20.0 to 21.0 Hz, more particularly 20.3 Hz; and 26.0 to 27.5 Hz, more particularly 26.4 Hz.

In some embodiments the MF may vary in a substantially continuous manner. Accordingly, the voltage or current signal will generally also be non-pulsed and continuously varying.

In addition to determining the current or voltage waveform, the amplifier 304 may also be configured to determine the length of time for which the system exposes the subject to the temporally-varying MF. For example, the MF may be produced only during a user-defined time period each day, for example a time period of 15 minutes to 1 hour per day. Alternatively, the system may be configured to produce the MF substantially continuously for a pre-determined period of time.

The respective amplitudes of the carrier signal and the amplitude modulation (also called amplitude variation) signal may be selected to produce a field having maximum MF intensity or flux density of 200 µT, according to ICNIRP recommendations 2010, in the target region in which the subject is expected to be located (e.g. regions 106A-C as shown in Figures 1A-C). In some embodiments a lower maximum magnetic field strength may be used as described herein, for example wherein the maximum magnetic field is 100 µT, 50 µT, 30 µT or 11 µT. The magnitude of the current or voltage signal required to produce such a MF is determined empirically for a particular configuration of coils and the final MF verified by frequency and Gauss meters. Furthermore, the positioning of the coils may be empirically determined and verified by control measures to produce a substantially homogenous MF intensity in the target region.

In embodiments, the respective amplitudes of the amplitude modulation signal and the carrier signal may be selected such that the peak amplitude of the amplitude-modulated signal, as illustrated in Figure 6, can be from 0% to 100% of the maximum amplitude. However, preferably the peak amplitude is at least 4 times the minimum amplitude. Suitably, the modulation index (i.e. the modulation signal amplitude / the carrier signal amplitude) is from 1 (i.e. 100% modulation ) to 0.6 (60% modulation), preferably the modulation index is from 80 to 100%, more preferably from 90 to 100% and most preferably about 100%. As will be well understood by the skilled person, an amplitude-modulated signal is produced by modulating a carrier signal (generally a sinusoidal waveform) by an amplitude modulation signal, thereby producing a waveform similar to that shown in Figure 6.

The MF exposure system as disclosed herein, for example the chair as illustrated in any one Figures 1A-C, Figure 4, or Figure 15, or the room or chamber as illustrated in Figure 2, or a bed, may be used to expose at least a part of a subject to the herein disclosed low frequency MF that varies according to an amplitude-modulated signal with a carrier frequency within the range of 400 to 450 Hz and a MF strength within the range of 0.5 to 200 µT (e.g. 10 to 200 µT). More particularly, the room or chamber as illustrated in Figure 2, may be used to expose at least a part of a subject to the herein disclosed low frequency MF that varies according to an amplitude-modulated signal with a carrier frequency within the range of 400 to 450 Hz, a modulation frequency within the range of 3 to 30 Hz, such as a modulation frequency corresponding to a Schumann resonance mode, and a MF strength within the range of 0.5 to 200 µT, e.g. 10 to 200 µT).

The Examples herein demonstrate that the modulated low frequency MF described herein enhances stem cell and progenitor cell (e.g. human epidermal stem and progenitor cell) proliferation and survival. The herein disclosed MF exposure system is therefore believed to be useful for reducing skin aging, for enhancing tissue regeneration, for enhancing wound healing, treating cancer and/or for enhancing immune function in a subject by exposing at least a part of the subject to said low frequency MF.

The modulated low frequency magnetic field (LF-MF) exposure system may be used for any of the methods or uses described herein. The system may be for use in enhancing *in-vitro* proliferation of stem and progenitor cells or other primary cells. The system may be for use in enhancing *in-vivo* proliferation of stem and progenitor cells. The system may be for use in the cosmetic treatment of a condition associated with skin stem cells or skin progenitor cells in a subject. The system may be for use in reducing skin aging. The system may be for use in the method for treating a medical condition associated with stem cells or progenitor cells in a subject. The system may be for use in enhancing tissue regeneration. The system may be for use in enhancing wound healing. The system may be for use in enhancing tissue regeneration. The system may be for use in the prevention or treatment of a cancer. The system may be for use in the method for inhibiting cell senescence. The system may be for use in the method for treating a senescence associated disease in a subject. The system may be for use in the treatment of a condition selected from an autoimmune disease, a neurodegenerative disease, an inflammatory disease, a cognitive disorder, and a psychiatric disorder.

Methods of treatment performed on a human or animal body as well as uses of a device for such treatments are not a part of the invention.

### Cell Proliferation

The MF exposure system, methods and uses disclosed herein are useful for enhancing *in vitro* proliferation of organic cells, particularly stem cells, progenitor cells or other primary cells, more particularly human epidermal stem and progenitor cells by exposing the cells to said LF-MF.

Stem cell and progenitor cell proliferation and differentiation are essential for tissue homeostasis and tissue regeneration, for example in injury or wound healing. Stem/progenitor cell activation, proliferation, maintenance and differentiation can become dysregulated in certain diseases and conditions, particularly age-related conditions. The modulated LF-MF described herein provide beneficial effects on stem and progenitor cells, for example by enhancing proliferation, reducing stem/progenitor cell aging and/or reducing stem/progenitor cell stress. Accordingly, exposing stem cells and progenitor cells to the modulated LF-MF described herein is expected to be beneficial in many therapeutic and non-therapeutic/cosmetic applications, including those described above in the brief description of the invention. In particular the modulated LF-MF is expected to be beneficial in the treatment or prevention of conditions associated with keratinocyte stem and progenitor cells.

### Wound Healing

The system, methods and uses of the modulated LF-MF described herein are expected to be beneficial in wound healing.

Wound healing is a complex and dynamic process involving, amongst other parameters interactions between dermal and epidermal dermal cells. Shortly after a wound is incurred, typically 2 to 10 days after wounding keratinocyte proliferation is increased and the formation of a continuous keratinocyte monolayer is rate limiting for successful wound healing (Harding, BMJ. 2002 Jan 19; 324(7330): 160-163). The increased proliferation of keratinocyte stem and progenitor cells exposed to the modulated LF-MF described herein is therefore expected to be beneficial in this process. For example, the modulated LF-MF may increase wound closure rate and hence prevent or reduce wound contracture, prevent or reduce scarring and/or enhance tissue re-modelling and finally reduce the time required for wound healing. It might also stimulate the rare event of hair follicle neogenesis (Ito, M., Nature. 2007 May 17; 447(7142): 316-320).

The modulated LF-MF may be applied to substantially all of the subject to promote wound healing. However, preferably the modulated LF-MF is applied locally to the wounded region, thereby exposing cells and cellular tissue at and close to the site of a wound to the modulated LF-MF described herein.

In certain embodiments wound healing may be treated by locally applying to a wound stem or progenitor cells that have been cultured ex *vivo* according to a method described herein, wherein stem cell and progenitor cells are exposed ex *vivo* to the modulated LF-MF to enhance proliferation and optionally differentiation of the cells. The cells may be applied directly to the wound site, for example by topical application or by injection. The cells may for example be applied to the wound using a suitable wound dressing or patch coated or impregnated with the cells. The cells may also be introduced into a wound together with a scaffold or matrix (e.g. a biodegradable polymer matrix) to further promote tissue regeneration in the wound. The scaffold of matrix may be impregnated or coated with the stem/progenitor cells. It is also possible to culture the stem/progenitor cells in the presence of the scaffold or matrix and the modulated LF-MF, thereby promoting growth of the cells directly on or within the scaffold or matrix.

The systems, methods and uses that utilise the modulated LF-MF described herein are expected be beneficial in promoting wound healing in a broad range of tissue and is not limited to epidermal wounds. For example the systems, methods and uses described herein that utilise the modulated LF-MF described herein may be useful for promoting wound healing (e.g. surgical wounds, injuries or other trauma) in tissues selected from muscle tissue, connective tissue, joint tissue, epithelial tissue, endothelial tissue, nervous tissue, fat tissue, skin tissue, lung tissue, liver tissue, bladder tissue, kidney tissue, cardiac tissue, pancreatic tissue, pancreatic tissue, stomach tissue, intestinal tissue, spinal tissue, brain tissue, eye tissue, fibrous tissue, dentin, bone or bone marrow. Thus in certain embodiments the systems, methods and uses described herein may be for promoting wound healing in a subject that has undergone neurosurgery, wherein the subject is exposed to the modulated LF-MF described herein to promote healing of brain tissue or nerve tissue. Suitably in this embodiment when the subject has undergone brain surgery, the head of the subject is exposed to the modulated LF-MF described herein. Also contemplated is localised application of the modulated LF-MF, wherein a particular part of the brain or nerve tissue is exposed to the modulated LF-MF.

### In Vitro Cell Culturing

As discussed in the brief description of the invention, cells, particularly stem cells, progenitor cells or other primary cells may be cultured *in vitro* wherein the cells (e.g. step or progenitor cells are exposed to the modulated LF-MF during culturing and expansion of the cell population.

*In vitro* cell culture methods and suitable culture media are well known to the skilled person and are illustrated in the Examples herein. Cell culture media suitable for the proliferation and/or differentiation of stem or progenitor cells are well known (Dakhore, S. et al., Human Pluripotent Stem Cell Culture: Current Status, Challenges, and Advancement. Stem cells international, 2018, 7396905). For example, the expansion medium may be a modified basal culture medium based on minimum essential medium (MEM), Dulbecco's modified Eagle's medium (DMEM) or minimum essential medium Eagle alpha modification (α-MEM). The stem cell expansion medium is suitable a serum-free stem cell expansion medium. Stem cell expansion media are commercially. For differentiation of the stem or progenitor cells a differentiation medium may be used, alternatively the expansion medium is supplemented with growth factors to promote differentiation of the stem or progenitor cells.

The cells are suitably cultured and grown in a suitable culture vessel. The cell culture cavity is a volume within the cell culture vessel that contains the cells to be proliferated, for example a cell culture medium comprising the cells. In use the cells contained within the cell culture cavity are exposed to the modulated LF-MF described herein.

The culture vessel may be any vessel suitable for the culture of stem or progenitor cells, for example a 2D tissue culture vessel or a 3D suspension culture vessel. In some embodiments the culture vessel is selected from a petri-dish, a multi-well plate, stacked cell culture vessel, a conical tube, or a 3D-suspension culture vessel.

In 2D culture vessels the vessels are typically coated with a suitable protein such as cadherin, laminin or vitronectin. The stem cells attach to the coated surface and grow in colonies on the surface. The cells are passaged before the colonies become too dense, for example when reaching greater than 50%, 60%, 70%, 80% or 90% confluence, and/or when the cells exhibit increased differentiation. Upon reaching the desired confluence the cells are disassociated from the surface of the vessel using a suitable method (e.g. shaking, mechanical scraping or enzymatic disassociation (e.g. trypsin-EDTA)). The collected cells are then re-seeded at a lower surface density in a vessel together with a culture medium to continue growth. The passaging may be performed numerous times to provide the required population of cells.

In 3D culture vessels the cells are suspended in a suitable culture medium and may form aggregates which are typically spherical as a suspension in the culture medium. 3D culturing can also be performed using a suitable gel or scaffold in which the cells are suspended. In 3D culture vessels the cells are passaged when the average size of the cell aggregates reaches 100 µm to 500 µm, and/or when the cells reach 50%, 60%, 70%, 80% or 90% confluence, and/or when the cells exhibit increased differentiation. The cell aggregates are collected and dissociated (e.g. using a suitable enzyme) and are diluted to the required density/concentration for continued growth.

In certain embodiments the cells are passaged multiple times whilst being exposed the modulated LF-MF as described herein. Thus it may be that the cells (e.g. stem or progenitor cells are passaged at least 2 times, at least 3 times, at least 4 times, at least 5 times , at least 6 times, at least 7 times or at least 8 times. For example the cells may be passaged 3 to 8 times or 3 to 6 times, wherein the cells are exposed to the modulated LF-MF during each passage.

Proliferation of the cells according to the methods described herein may be monitored using conventional methods, for example optical microscopy to determine the degree of confluence.

Stem and progenitor cells and differentiated cells produced according to the method described herein may also be used to develop *in-vitro* models of tissue for *in vitro* modelling of diseases and the testing and screening drug candidates for efficacy and/or toxicity. Such cellular models or "diseases in a dish" have been used to study cardiomyocytes for cardiac disease models, and the modelling of neurological disorders.

### Treatment of Autoimmune Disease, Neurodegenerative Disease, Inflammatory Disease, Cardiovascular Disease, Cognitive Disorders and Psychiatric Disorders

As set out in the brief description of the invention the review by Guerriero et al., suggests that low frequency magnetic fields may be beneficial in the treatment of a number of conditions, including autoimmune disease, neurodegenerative disease, inflammatory disease, cognitive disorders, psychiatric disorders, spinal injuries, ocular diseases.

The modulated LF-MF described herein may be for the treatment of a neurodegenerative disease, for example selected from Alzheimer's disease; amyotrophic lateral sclerosis; motor neurone disease; motor disorders; Parkinson's disease; Huntington's disease, or multiple sclerosis.

The modulated LF-MF described herein may be for the treatment of cognitive impairment, for example cognitive impairment associated with any of the neurodegenerative diseases above.

The modulated LF-MF described herein may be for the treatment of a disorder selected from a mood disorder, post-traumatic stress syndrome, attention deficit disorder attention deficit hyperactivity disorder, depression, or a depressive disorder.

The modulated LF-MF may be suitable for the treatment of bullous diseases of the skin such as autoimmune diseases of the pemphigus complex or inherited diseases like Epidermolysis simplex.

The modulated LF-MF may be suitable for the treatment of spinal cord injuries. For example to regenerate new nerve cells and promote growth of new nerve fibres and/or to improve nerve function.

The modulated LF-MF may be suitable for the treatment of ocular diseases, for example to regenerate damage to cornea, retinal optic nerve, treatment of age-related macular degeneration (AMD), glaucoma or retinitis pigmentosa.

The modulated LF-MF may be suitable for the treatment of autoimmune disorders, for example the treatment of type 1 diabetes to replenish pancreatic β-cells; or the treatment of multiple sclerosis (e.g. to regenerate neurons with a myelin sheath).

The modulated LF-MF may be suitable for the treatment of a cardiovascular condition, for example to regenerate cardiac muscle, for example resulting from cardiac ischemia; or to restoring cardiac blood vessels.

### Modulation of Brain Waves

Electroencephalogram (EEG) studies indicate that brain waves are divided into five different bandwidths. Alpha-band waves have a frequency of 8.5 to 12.5 Hz and are known to reduce psychological stress and induce a relaxed mood state, increased attention and improve memory (Klimesch, W. alpha-band oscillations, attention, and controlled access to stored information. Trends Cogn. Sci 16, 606-617, doi:10.1016/j.tics.2012.10.007 (2012); Hammerschlag, Biofield Physiology: A Framework for an Emerging Discipline., Glob Adv Health Med, 4, 35-41, doi:10.7453/gahmj.2015.015.suppl (2015)).
Alpha-band waves also increase serotonin production and reduce cortisol levels boosting the immune system (Yu, Activation of the anterior prefrontal cortex and serotonergic system is associated with improvements in mood and EEG changes induced by Zen meditation practice in novices. Int J Psychophysiol 80, 103-111, doi:10.1016/j.ijpsycho.2011.02.004 (2011); Puig, M. V. & Gulledge, A. T. Serotonin and prefrontal cortex function: neurons, networks, and circuits. Mol Neurobiol 44, 449-464, doi:10.1007/s12035-011-8214-0 (2011); and Lozano-Soldevilla, D. On the Physiological Modulation and Potential Mechanisms Underlying Parieto-Occipital Alpha Oscillations. Front Comput Neurosci 12, 23, doi:10.3389/fncom.2018.00023 (2018)).

Beta-band waves have a frequency of 12.5 to 30 Hz and are associated with alertness, focus and cognition. The Examples herein show that both alpha and beta-band waves are increased when subjects were exposed to the modulated LF-MF described herein. Subjects undergoing the tests described an increased state of relaxation, which is consistent with increased alpha-band activity. Normally alpha-band waves are not detected when the eyes are open. Surprisingly it was found that in subjects exposed to the modulated LF-MF, alpha-band activity was also increased and persisted in particular in the Parietal Occipital cortex, even with the eyes open.

The observed increase in both alpha-band and beta-band activity resulting from exposure to the modulated LF-MF suggest that the modulated LF-MF may be useful in reducing anxiety and stress and/or increasing alertness, and/or increasing focus and/or enhancing cognition. In a particular embodiment the LF-MF is used for the reduction of physiological stress.

The modulated LF-MF described herein may therefore be useful in the treatment or prevention of an attention deficit disorder (e.g. attention deficit hyperactivity disorder (ADHD)), post-traumatic stress syndrome, cognitive impairment, or and anxiety disorder.

In some embodiments the modulated LF-MF is for use in the treatment or prevention of an anxiety disorder. Anxiety is a feeling of apprehension or fear that lingers due to an individual's perception of persistent and unrelenting stress. Anxiety is typically accompanied by various physical symptoms including twitching, trembling, muscle tension, headaches, sweating (e.g., night sweats), dry mouth, or difficulty swallowing. Examples of anxiety disorders include separation anxiety disorder, selective mutism, specific phobia, social anxiety disorder (social phobia), panic disorder, panic attack, agoraphobia, post-traumatic stress disorders (PTSD), generalized anxiety disorder, substance/medication-induced anxiety disorder, anxiety disorder due to another medical disorder or an obsessive compulsive disorder.

Additionally, the examples herein show that exposure of cells to the modulated LF-MF reduced markers for cell stress and cell aging, suggesting that the LF-MF will provide beneficial effects in subjects exposed to the modulated LF-MF.

Furthermore, the herein disclosed MF exposure system is believed to be useful for improving wellbeing, for reducing stress, for enhancing concentration and/or for reducing anxiety in a subject by exposing at least a part of the subject to said LF-MF. The system may be for use in non-therapeutic improvement of well being, reducing stress, including psychological stress, inducing relaxation, enhancing concentration, increasing focus, increasing alertness, enhancing cognition add/or reducing anxiety.

Thus also provided is a non-therapeutic method for improvement of well being, reducing stress, including psychological stress, inducing relaxation, enhancing concentration, increasing focus, increasing alertness, enhancing cognition add/or reducing anxiety in a subject, the method comprising exposing at least a part of the subject to the LF-MF.

In the embodiments herein relating to the modulation of brain-waves, it is preferred that at least the brain of the subject is exposed to the LF-MF.

### Examples:

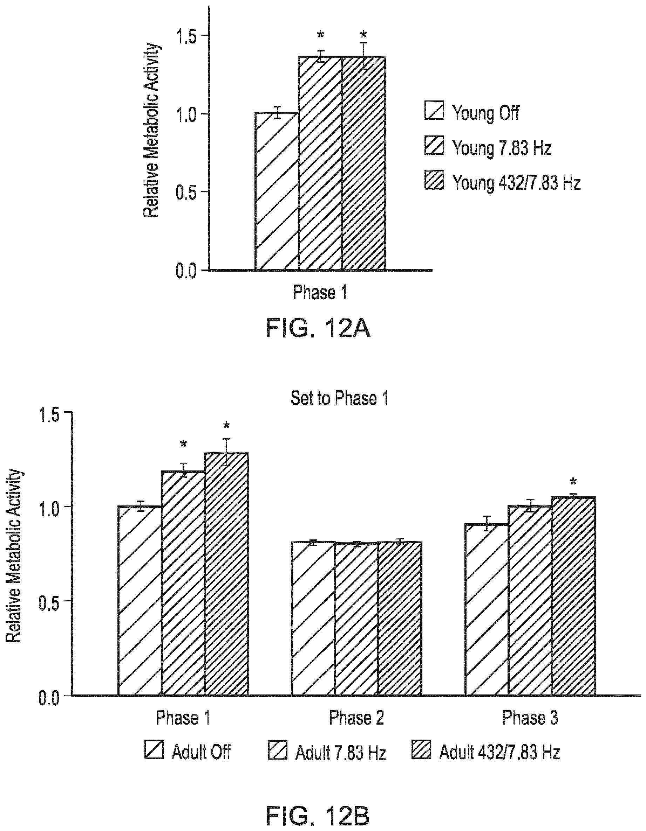

Unless explicitly described otherwise, the following applies:
- Pooled young (below the age of 8; 3 donors) or adult primary human stem and progenitor keratinocytes (major cell type in the epidermis) were seeded in 6-well plates at an initial cell density of 4,000 cells /cm² containing 2 ml CnT-07 (CnT-BM.1 (basal cell media with low calcium concentration (0.07 mM)) supplemented with CnT-07.S).
- Pooled young primary human fibroblasts (major cell type found in the dermis) were seeded in 6-well plates at an initial cell density of 2,000 cells /cm² containing 2 ml CnT-Prime F.
- All cells, media and complementary products were purchased from CELLnTEC Advanced Cell Systems AG (Switzerland).
- The culture media were free from antibiotics or antimycotics and were changed every 2 to 3 days.
- The cells were cultured at 37 °C ± 0.1 °C, 90 % ± 5% humidity in 5 % ± 0.1% CO₂.
- All groups contain at least 3 or more sample values (at least triplicates) from 3 or more independent experiments.
- Upon any group reaching 80 % confluency (Phase 1, described in Figure 5), all cells from all group were harvested unless when investigating metabolic activity (Figure 12).
- Cells were detached using CnT-Accutase to count and passage the cells.
- Live and dead cells (for proliferation and survival, respectively) were counted using trypan blue and the apparatus "Countess II", supplied by Thermo Fisher.
- Cells were exposed to magnetic fields as described in each example using a classical Helmholtz coil (Radius: 15 cm ) cast into the incubators and fed by a generator and amplifier.
- All treatment conditions started on the same day of seeding.
- All references to "coil Off" or "Off" indicate cells were incubated in the coil without electrical connection. Results of "coil off" and no coil were indistinguishable (data not shown).
- The MF exposure system was configured to produce 7.83 Hz (320 mV), 432 Hz (570 mV) or a 432 Hz driver frequency with 7.83 Hz amplitude modulation (1050 mV) at an average intensity of 200 µTesla (in compliance with ICNIRP 2010 guidelines). Figure 6A illustrates the wave-form of the 432 Hz driver/carrier with 7.83 Hz 100% amplitude modulation frequency (herein also referred to as "432/7.83 Hz" and the "combined MF") as used in the study.
- The MF exposure system was configured and continuously tested to generate pure and stable fields (± 2% variations) over the entire period of the experiments with incubators set at 37 °C and 90 % humidity (± 2% variations).
- Metabolic turnover of cells in Phase 1-3 (described in Figure 12) was measured using a colorimetric assay based on conversion of a MTS tetrazolium compound into a coloured formazan dye by NAD(P)H-dependent dehydrogenase in metabolically active cells (MTS assay) according to the protocols of the provider (abcam).
- A quantitative PCR-based assay was used to determine telomere length after MF exposure as described by Cawthon, R. M., "Telomere measurement by quantitative PCR", 10.1093/nar/30.10.e47, may 2002).
- All results are shown as mean ± SEM.
- For normalized results the coil Off group was set to 1.
- Statistical analyses: When comparing data between two groups, an unpaired 2-tailed Student's t test was used. When comparing data between three or more groups, an unpaired one-way ANOVA followed by Tukey's multiple comparisons test were used. Analyses were performed using the GraphPad Prism 8 software. Asterisks denote statistical significance (*P < 0.05; **P < 0.01; and ***P < 0.001).
- Cell morphology based on cell size, cell shape and granularity was assessed blindly through visual inspection and picturing via light microscopy by two experienced researchers.

Figure 5 illustrates the three main maturation phases involved in epidermal homeostasis in vivo and in vitro. In phase 1 stem and progenitor keratinocytes proliferate, in phase 2 when the keratinocytes become confluent they exit the cell cycle (G0) and commit to differentiation in phase 3 (adapted from Kolly, C.et al, J Invest Dermatol 124, 1014-1025 (2005)).

Keratinocytes in the stratum basale intermittently proliferate, exit the cell cycle and go on to differentiate while migrating to the suprabasal layer during skin homeostasis.

Key events at cell cycle exit involve the suppression of c-Myc, which in turn sets the starting point for onset of differentiation. Consequently, expression of differentiation markers like keratin 10, Dsg1, involucrin and loricrin increase in cultured cells and in the epidermis during the terminal differentiation (Y. Poumay el al, J Invest Dermatol 104:271-276 (1995); Kolly, C.et al, J Invest Dermatol 124, 1014-1025 (2005)). The increase of extracellular calcium (switch) accelerates the process.

Under standard conditions, keratinocytes stop proliferating at 100% confluency following contact inhibition and cell cycle exit. In contrast, uncontrolled growth overriding contact inhibition is a hallmark of cancer and other hyper proliferative diseases.

### Example 1: Effect of 432/7.83 Hz MF on stem cell and progenitor cell proliferation

Primary young and adult donor keratinocytes were seeded and then exposed in a coil without generated MF (coil Off) or 432/7.83 Hz MF.

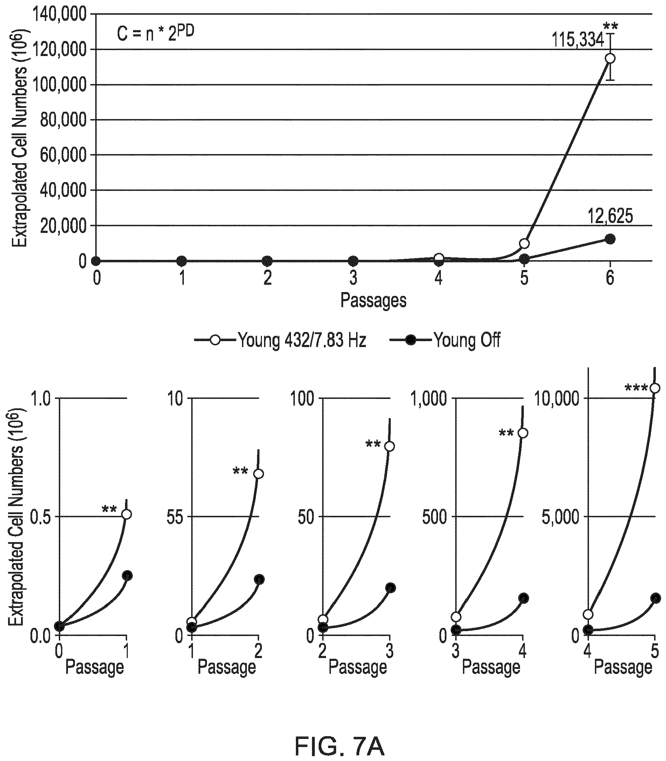

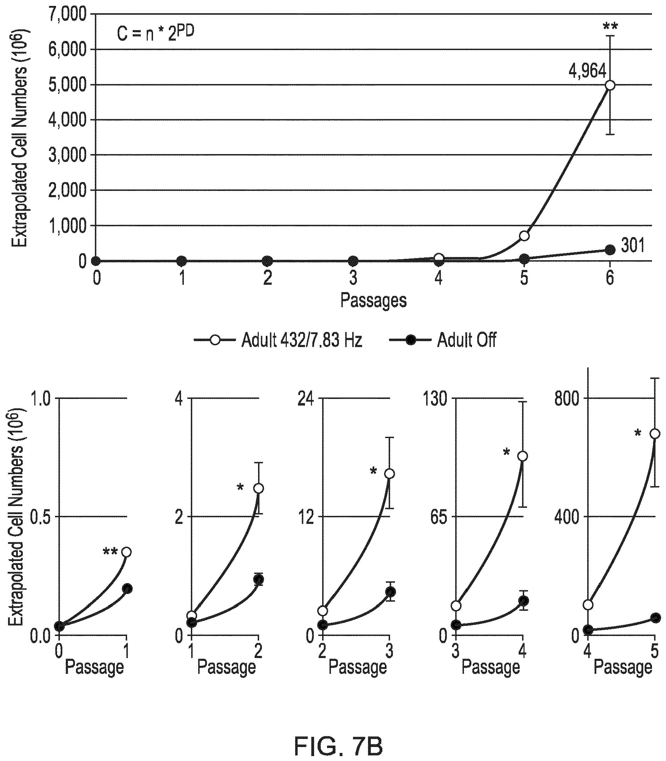

This experiment was continued for a total of 6 passages to compare short-term and long-term effects of LF-MF on cell proliferation. Extrapolated cell numbers (Figure 7) were calculated as follows: C = n * 2 ^{PD}, where "C" is the calculated cell number, "n" is the number of cells seeded, "PD" is the cumulative population doubling for each respective passage. "PD" is calculated as 3.33*(LOG(Cell collected/Cells seeded)).
The significant increase in cell count for primary young (Figure 7A) and adult (Figure 7B) donor keratinocytes exposed to 432/7.83 Hz MF remained constant for each passage over the whole time-course of the experiment (Figure 7).

### Example 2: Effect of 432/7.83 Hz modulated MF compared to 7.83 Hz alone

Primary young and adult donor keratinocytes were seeded and then exposed to coil Off or 7.83 Hz or 432/7.83 Hz MF.

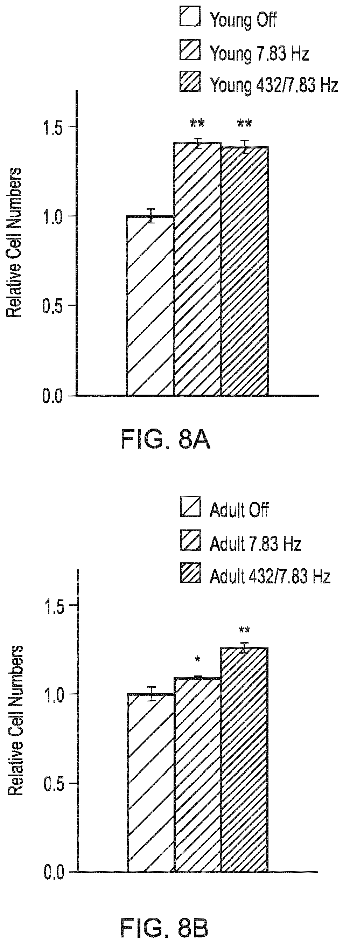

After one passage of continuous MF exposure, a significant increase in the number of young cells (Figure 8A) was observed in both 7.83 Hz and 432/7.83 Hz groups compared to coil Off groups.

The effects of continuous MF exposure were investigated in parallel on primary adult donor keratinocytes after one passage (Figure 8B).

As observed with the primary young cells (Figure 8A), the number of adult cells was significantly increased in both 7.83 Hz and 432/7.83 Hz groups compared to Off MF (Figure 8B). Interestingly, the 432/7.83 Hz exposed adult keratinocytes proliferated significantly better than 7.83 Hz exposed cells.

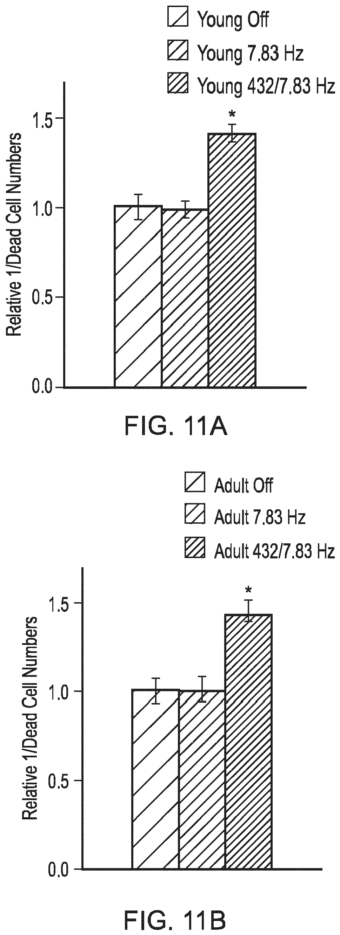

Furthermore, similar to the primary young donor cells, the 432/7.83 Hz exposed cells had the additional beneficial effect of increasing the cell survival rate on average by 45% (see Figure 11).

Regarding morphology, the adult donor keratinocytes also exhibited improvements when exposed to 7.83 Hz and 432/7.83 Hz, with the healthiest morphology seen in the 432/7.83 Hz exposed group.

The results in primary adult donor cells corroborated the observations in primary young donor cells, that the 432/7.83 Hz MF may increase cell proliferation when compared to the 7.83 Hz and Off groups.
Figures 8A-B demonstrate improved growth of primary human stem and progenitor keratinocytes in Phase 1 when the cells are exposed to the 432/7.83 Hz MF for 1 passage.

### Example 3: 432/7.83 Hz modulated MF compared to non-modulated driver frequency of 432 Hz alone on stem cell and progenitor cell proliferation

Primary young donor keratinocytes were seeded and then exposed to either coil Off, 432 Hz or 432/7.83 Hz MF.

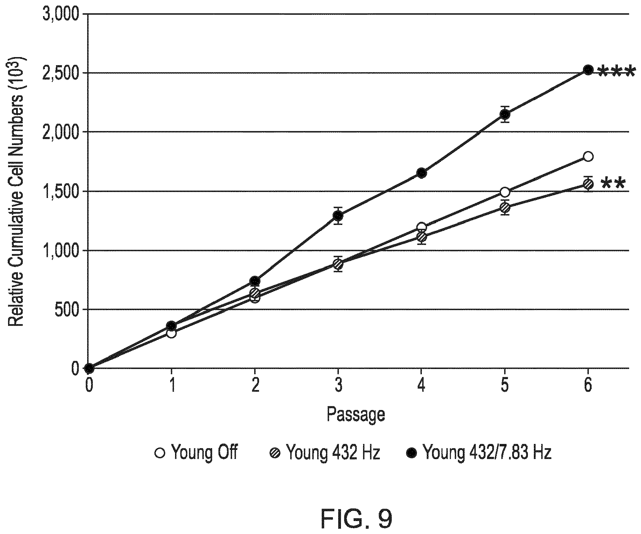

After 1 passage, the 432Hz exposed keratinocytes exhibited a significantly higher cell count than the coil Off control group. However, the 432/7.83 Hz MF group exhibited higher proliferation (Figure 9).

Over the first two passages, there was a small increase in cell count for the 432 Hz driver frequency exposed group, however, this turned into a negative effect after the third passage and the number of live cells started to decline in this group. After 6 passages of exposure cells in the 432Hz showed a significant decrease in cell count compared to the coil Off control group. In contrast cells in the 432/7.83 Hz MF group showed a significant increase in proliferation compared to the coil Off control (Figure 9).

These results show that the modulated 432/7.83 Hz MF results in higher proliferation of primary young donor keratinocytes compared to cells exposed to the 432 Hz driver frequency alone.

### Example 4: Effects of modulation frequency, driver frequency and magnetic field intensity on stem and progenitor cell proliferation

Primary young and adult donor keratinocytes were seeded and then exposed to the following MF conditions to assess the effects of modulation frequency, driver frequency and magnetic field intensity on proliferation.

### Example 4.1: Effects of modulator frequency on proliferation.

Cells were exposed to the following conditions during cell culturing:
Coil Off (control)
432 Hz amplitude modulated at 3 Hz
432 Hz amplitude modulated at 7.83 Hz
432 Hz amplitude modulated at 10 Hz
432 Hz amplitude modulated at 30 Hz

For primary young donor keratinocytes, modulation frequencies of 432/30 Hz, 432/50 Hz and 432/100 Hz resulted in a significant increase in proliferation compared to the control (coil Off) (Figure 10A). A very significant rate of proliferation was observed in young donor cells exposed to 432/7.83 Hz. In adult keratinocytes a significant increase in proliferation was observed in cells exposed to 432/7.83 Hz. A non-significant increase in proliferation was observed at 432/30 Hz. Reduced proliferation occurred in the groups exposed to 432/50 Hz and 432/100 Hz (Figure 10B).

### Example 4.2: Effects of driver frequency on proliferation.

Cells were exposed to the following conditions during cell culturing:
Coil Off (control)
368 Hz amplitude modulated at 7.83 Hz
400 Hz amplitude modulated at 7.83 Hz
432 Hz amplitude modulated at 7.83 Hz
464 Hz amplitude modulated at 7.83 Hz

Significant increases in proliferation were observed in the primary young donor keratinocytes exposed to 368/7.83 Hz and 400/7.83 Hz. A highly significant increase in proliferation was observed in the primary young donor cells exposed to 432/7.83 Hz. A non-significant effect was observed in the 464/7.83 Hz group (figure 10C).

In primary adult donor keratinocytes significant increases in proliferation were observed in the 400/7.83 Hz and 432/7.83 Hz groups. (Figure 10D).

### Example 4.3: Effects of magnetic field intensity on proliferation.

Cells were exposed to the following conditions during cell culturing:
Coil Off (control)
10 µT, 432 Hz amplitude modulated at 7.83 Hz at 55 mV pp
30 µT, 432 Hz amplitude modulated at 7.83 Hz at 550 mV pp
200 µT, 432 Hz amplitude modulated at 7.83 Hz at 1050 mV pp
250 µT, 432 Hz amplitude modulated at 7.83 Hz at 1250 mV pp

Significant increases in proliferation were observed in all groups in the primary young donor keratinocytes compared to control cells (Figure 10E). In the primary adult donor keratinocytes significant increases in proliferation were observed in cells exposed to 30 µT and 200 µT (Figure 10F).

### Example 5: Effects of MF treatment on cell survival

The same primary young and adult donor keratinocytes described in Example 8 below were seeded and then exposed to coil Off or 7.83 Hz or 432/7.83 Hz MF.

After one passage of continuous MF exposure to 432/7.83 Hz the survival rate the of young cells was significantly increased (Figure 11A).

The keratinocytes also displayed better morphology in both 7.83 Hz and 432/7.83 Hz groups, with the healthiest looking cells observed in the 432/7.83 Hz group.

Similar to the young donor cells, the 432/7.83 Hz exposure significantly increased the survival rate of adult cells (Figure 11B).

The primary adult donor keratinocytes also exhibited improvements in cell morphology when exposed to 7.83 Hz and 432/7.83 Hz frequencies, with the healthiest morphology seen in the 432/7.83 Hz group.

The results in adult donor cells corroborated the observations in primary young donor cells, that the 432/7.83 Hz MF significantly improves cell survival rate when compared to the 7.83 Hz and coil Off groups.
Figures 11A-B demonstrate improved survival rate of primary human stem and progenitor keratinocytes in Phase 1 when the cells are exposed to the 432/7.83 Hz MF for 1 passage.

### Example 6: Effects of MF treatment on contact inhibition

A high throughput assay was set up to measure cell numbers based on metabolic activity (MTS) throughout Phase 1 (80% confluency), Phase 2 (100% confluency) and Phase 3 (5 days after 100% confluency was reached and the calcium concentration was increased to 1.2 mM) (Figure 5). If contact inhibition occurs in Phase 2, cells are expected to stop proliferating while metabolic activity decreases and becomes similar across all treatment groups.

Growth correlates with metabolic activity and was assessed by MTS assay.

Primary young and adult donor keratinocytes were seeded and then exposed to coil Off or 7.83 Hz or 432/7.83 Hz MF.

Figure 12 shows primary young keratinocytes in Phase 1 after one passage (Figure 12A) and primary adult keratinocytes across all maturation states (Phase 1, 2 and 3; Figure 5) (Figure 12B). Graphs represent substrate conversion relative to coil Off MF in Phase 1 set to 1.

Significant increases in metabolic activity in Phase 1 of primary young donor keratinocytes exposed to 432/7.83 Hz or 7.83 Hz MF and in adult keratinocytes exposed to 432/7.83 Hz MF were observed after one passage (Figure 12A and 12B).

When the time course was continued into Phase 2 and 3 by letting the cells become confluent (Phase 2) and enter differentiation (Phase 3), a reduction in the metabolic turnover in Phase 2 and no difference between the treatment groups (Figure 12B, shown for adult donor keratinocytes) were observed. In Phase 3, when the cells start to differentiate, metabolic activity again slightly increased.

Taken together the results shown in Figures 12A-B demonstrate that primary human stem and progenitor keratinocytes treated using 432/7.83 Hz MF exhibited normal contact inhibition and metabolic turnover, thus suggesting that tumorigenic overgrowth does not occur.

### Example 7: Efficacy of MF treatment on senescence

Telomere shortening is a readout of senescent cells (Zhao et al., Telomere length maintenance, shortening, and lengthening. J. Cell Physiol. 229, 1323-1329 (2014). Cell senescence (cessation of cell division) is believed to be implicated in a number of diseases and medical conditions, for example carcinogenesis, aging and tissue repair.

The number of telomere repeats in human stem and progenitor keratinocytes was measured using quantitative PCR using a method analogous to that described in Cawthon, R.M. Telomere measurement by quantitative PCR. Nucleic Acids Res 30, e47 (2002). This approach represents the most frequently used method to assess the number of repeats reflecting telomere length.

In this experiment, it was also observed that different stocks of keratinocytes from the same donor and passage may exhibit different reactivity to 432/7.83 Hz MF in Phase 1. Without being bound by theory it is thought that there may be some initial variability in responsiveness to the MF associated with the initial thawing of a sample.

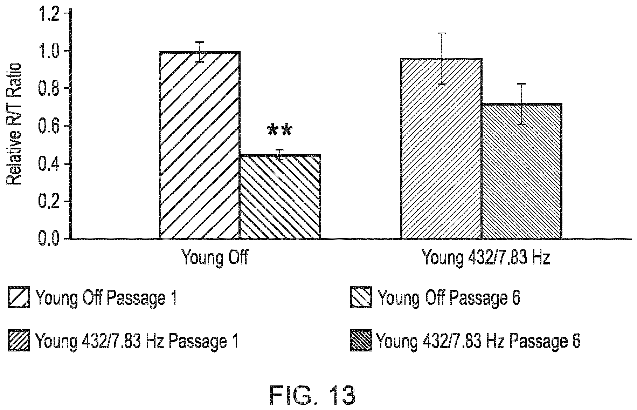

To further assess the effect of 432/7.83 Hz MF on telomere length, early (Passage 1) and late time-points (Passage 6) were compared. Using an analogous method to that described in Example 1 young donor pooled keratinocytes were lysed after passage 1 and after passage 6 in Phase 1 and DNA isolated. After 6 passages, the control (Off) featured a decrease in telomere length of more than 50% compared to the first passage. In contrast, the decrease in telomere length for the 432/7.83 Hz MF treated keratinocytes was not significant (Figure 13).

These results suggest that the 432/7.83 Hz MF treatment provides protective effect on telomere length.

### Example 8: Effect of MF on cellular stress

Primary young donor keratinocytes were seeded, then cultured in either normal medium (CnT-07) or starved medium (25% CnT-07, 75% PBS), and exposed to coil Off or 432/7.83 Hz MF.

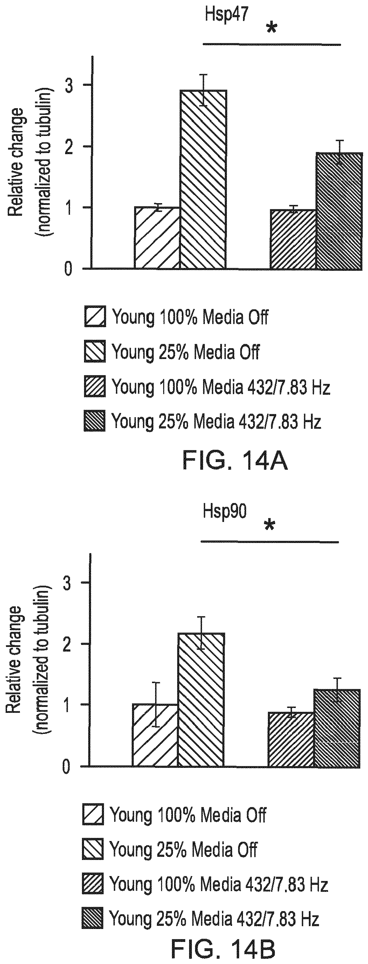

All samples were harvested for In-Cell Western analysis, staining for heat shock protein 47 (Hsp47) and 90 (Hsp90), both being stress markers (Scieglinska et al., Heat shock proteins in the physiology and pathophysiology of epidermal keratinocytes; Cell Stress Chaperones 24, 1027-1044 (2019)).
The group cultured in 25% media without exposure to MF (Off) displayed an approximately three-fold increase in relative Hsp47 expression level compared to cells cultured in the 100% medium. Hsp90 expression was also increased compared to the 100% medium. In contrast Hsp47 (Figure 14A) and Hsp90 (Figure 14B) expression in the modulated 432/7.83 Hz MF exposed groups were significantly reduced compared to the unexposed controls (coil Off). In figure 14 the relative Hsp47 and Hsp90 expression was normalised to β-tubulin, routinely used as housekeeping protein control to normalize the levels of protein detected.

### Summary of Examples 1 to 8

These studies demonstrated that the 432/7.83 Hz amplitude-modulated MF has a significant effect on enhancing human epidermal stem and progenitor cell proliferation and survival. The effect on proliferation of human epidermal stem and progenitor cells was shown to be superior to the use of 7.83 Hz alone, the use of 432 Hz alone, varied amplitude modulation frequencies, varied driver frequencies and varied average field intensities.

Further, the 432/7.83 Hz amplitude-modulated MF showed additional advantages such as increasing cell survival across all epidermal maturation phases.
The use of 432/7.83 Hz amplitude-modulated MF, but not the use of 7.83 Hz alone, was further demonstrated to prevent telomere shortening. Telomere maintenance is a characteristic feature of stem cell-mediated tissue regeneration.

### Example 9: EEG Study

To assess the effect of modulated 432/7.83 Hz MF on the entrainment of brain waves, a one-blinded crossover pilot study was performed. In the study human brain states were investigated by measuring electroencephalogram (EEG) activity of subjects before and after 15 minutes of exposure to sham (no MF) or verum (432/7.83 Hz MF) in a chair wherein the subject was unaware of the exposure conditions and was seated in a cavity between Helmholtz coils located in the sidewalls of the chair such the head and substantially all the torso of the subject was exposed to the MF (Figure 15). To comply with ICNIRP 2010 recommendations, a field strength intensity of 30 µT was used.

A total of 4 healthy subjects (3 females, 1 male) were measured in the study in order to assess the potential of the modulated 432/7.83 Hz MF to systematically alter brain functional states (namely anxiety, stress and arousal) and of using multichannel resting state EEG to reliably detect these changes (Cantonal Ethics Committee Bern, approval Req-2020-00895).

The subjects were comfortably sat in an isolated room with adequate lighting and background noise. Blinded cross-over sessions were repeated multiple times (n = 18 sessions) with sham (no MF) or verum (432/7.83 Hz MF) conditions to compare 5 minutes before and 5 minutes after exposure, with both eyes closed and eyes opened at an interval of 30 seconds, to investigate the effect on brain wave activity. In detail, each EEG was recorded with 32 electrodes, positioned according to the international 10-10 system. All recordings were conducted using commercially available and safety certified systems (BrainVision Analyzer, Vers. 2.2.0, Brain Products GmbH, Gilching, Germany; recording reference FCz, analogue bandpass filter from 0.1 to 200 Hz, sampling rate 500 Hz).

The EEG data was then processed in a Brain Vision Analyzer. First, channels with bad signal, in terms of high noise to signal ratio, were excluded. Pre-processing of EEG data involved band pass filtering using a low cut-off of 0.1 Hz and a high cut-off of 30 Hz. An Independent Component Analysis (ICA) was then used to identify and eliminate eye movement components. The EEG was then visually inspected for residual artefacts and re-referenced to the average reference. Separately for the pre- and post-period and for eyes open and eyes closed, artefact free 2-second epochs were then extracted and submitted to an FFT. These single epoch FFT results were averaged within pre-defined frequency bands (Delta: 0.5 - 3.5 Hz, Theta 1: 3.5-5.5 Hz, Theta 2: 5.5 - 8.5 Hz, Alpha 1: 8.5 - 10.5 Hz, Alpha 2: 10.5 - 12.5 Hz, Beta 1: 12.5 - 18 Hz, Beta 2: 18 - 21 Hz and Beta 3: 21 - 30 Hz). The results were then averaged within conditions, i.e. separately for eyes open and eyes closed, both in the pre- and the post-stimulation periods and post and pre sham or verum values subtracted. The standard deviation of these means was also retained.

After one session of 15 minutes 432/7.83 Hz MF exposure, but not after sham, the subjects described an increased state of relaxation. This effect persisted after each following sessions.

Shown in Figure 16A are graphs depicting the average power spectrum of the different frequency bands "post" minus "pre" for each participant for eyes open and eyes closed during sham and verum. There is a clear increase in the upper alpha frequency band during the verum (solid curve) compared to sham condition.

To summarize the EEG data, t-maps averaged across all subjects for alpha 1, alpha 2 and beta 1 waves are shown in Figure 16B.These mean t-maps were calculated as "post" minus "pre" for each recording, which was then used to compare between conditions. From left to right are verum eyes closed (EC), sham eyes closed, verum eyes open (EO) and sham eyes open.

The alpha 1 waves increased during verum compared to sham in EC, this was also the case in EO. The alpha 2 waves also increased during verum compared to sham in both EC and EO. Both alpha 1 and 2 increased activities in verum indicates reduced anxiety and stress. The beta 1 waves increased in verum EO and not EC, indicating increased alertness and focus.

Normally alpha waves are mostly present during EC as EO suppresses their activity. Interestingly the verum increases alpha waves activity in both EC and even in EO, when alpha waves are normally not detected. This indicates that the alpha waves persist after 432/7.83 Hz MF to such a strong extent that even opening the eyes doesn't suppress their activity.

Taken together the results of the EEG study, when comparing multiple sessions across multiple days, the modulated MF exposure significantly increased both alpha and beta waves activities (Figure 16). This is expected to reduce anxiety and stress as well as increased alertness and focus in the subjects exposed to the modulated 432/7.83 Hz MF.

Studies were conducted with a field strength of 30 µT (in compliance with the ICNIRP 2010 recommendations).

## Claims

1. A magnetic field exposure system for exposing organic cells, cellular tissue or at least a part of a subject to a low frequency magnetic field, said system comprising a magnetic field generator comprising one or more coils configured to produce a magnetic field that varies according to an amplitude modulated signal with a carrier frequency of from 360 to 450 Hz, wherein the modulation frequency is from 0.5 to 100 Hz; and wherein the magnetic field has a field strength of from 0.5 to 250 µT.

2. The magnetic field exposure system according to claim 1, wherein said one or more coils are configured to at least partially surround the subject to be exposed to said low frequency magnetic field.

3. The magnetic field exposure system according to any of the preceding claims, wherein the system comprises a chair or bed configured such that at least part of a subject sitting in the chair or lying on the bed are exposed to the low frequency magnetic field.

4. The magnetic field exposure system according to claim 3, wherein the chair comprises a seat (1801) located in a cavity formed between two sidewalls (1802);
optionally wherein circular coils (1803) of the same diameter are located in each sidewall (1802), wherein the coils (1803) are located co-axially thereby forming a Helmholtz coil.

5. The magnetic field exposure system according to any one of claims 1 to 3, wherein the system comprises a bed and the one of more coils surround the bed or are located above and/or below the bed;
optionally wherein:
(i) the one or more coils surround the bed and are coaxially located along the horizontal axis of the bed; and/or
(ii) the one or more coils comprise a Helmholtz coil.

6. The magnetic field exposure system according to claim 1, wherein the system comprises a housing with the one of more coils located within the housing, wherein the housing is configured to be positioned to expose a specific body part or tissue of a subject to the low frequency magnetic field;
optionally wherein the one or more coils comprise a Helmholtz coil.

7. The magnetic field exposure system according to claim 1, wherein the system is in the form of a cell culture system, said system comprising cell culture vessel defining a cell culture cavity within the vessel and a magnetic field generator with one or more coils configured to produce a magnetic field within the cell culture cavity that varies according to an amplitude-modulated signal with a carrier frequency of from 360 to 450 Hz, wherein the modulation frequency is from 0.5 to 100 Hz; and wherein the magnetic field has a field strength of from 0.5 to 250 µT.

8. An *in-vitro* method for enhancing proliferation of organic cells by exposing the cells to a low frequency magnetic field that varies according to an amplitude-modulated signal with a carrier frequency of from 360 to 450 Hz, wherein the modulation frequency is from 0.5 to 100 Hz; and wherein the magnetic field has a field strength of from 0.5 to 250 µT;
optionally wherein:
(i) the cells are stem cells, progenitor cells or other primary cells;
(ii) the cells are stem cells or, progenitor cells;
(iii) the cells are human stem cells or human progenitor cells;
(iv) the cells are human keratinocyte stem cells or human keratinocyte progenitor cells;
(v) the cells are terminally differentiated primary cells; or
(vi) the cells are adult human stem cells selected from muscle stem cells, hematopoietic stem cells, epithelial stem cells, neural stem cells, mesenchymal stem cells, mammary stem cells, intestinal stem cells, mesodermal stem cells, endothelial stem cells, skin stem cells, melanocyte stem cells, hair follicle stem cells, olfactory stem cells, neural crest stem cells and dental pulp stem cells.

9. A non-therapeutic method for treating a condition associated with skin stem cells or skin progenitor cells in a subject, the method comprising exposing cellular tissue of at least a part of the subject to a low frequency magnetic field that varies according to an amplitude-modulated signal with a carrier frequency of from 360 to 450 Hz, wherein the modulation frequency is from 0.5 to 100 Hz; and wherein the magnetic field has a field strength of from 0.5 to 250 µT;
optionally wherein the condition associated with skin stem cells or skin progenitor cells is selected from maintaining skin homeostasis, reducing skin aging, enhancing skin rejuvenation, preventing or reducing skin wrinkles, increasing skin elasticity, preventing or reducing skin stretch marks, preventing or reducing cellulitis, increasing skin hydration, reducing skin roughness, reducing skin pore size, inducing hair growth, preventing or reducing hair loss, inhibiting hair greying, inducing hair re-pigmentation, maintaining skin pigmentation, and inducing nail growth.

10. A non-therapeutic method for improving wellbeing, and/or enhancing concentration in a subject by exposing at least a part of the subject to a low frequency magnetic field that varies according to an amplitude-modulated signal with a carrier frequency of from 360 to 450 Hz, wherein the modulation frequency is from 0.5 to 100 Hz; and wherein the magnetic field has a field strength of from 0.5 to 250 µT;
optionally wherein
(i) the method is a non-therapeutic method for inducing relaxation, enhancing concentration, increasing focus, increasing alertness, and/or enhancing cognition; and/or
(ii) the method increases alpha-band and/or beta-band activity in the subject.

11. The system or method according to any one of claims 1 to 10, wherein the magnetic field varies according to an amplitude modulated signal with a carrier frequency of from 400 to 450 Hz and a modulation frequency of 3 to 30 Hz;
optionally wherein:
(i) the carrier frequency is 432 Hz; and/or
(ii) the modulation frequency is from 3 to 28 Hz;
(iii) the modulation frequency corresponding to a Schumann resonance mode;
(iv) the modulation frequency is within the range of 7.50 to 8.00 Hz;
(v) the modulation frequency is 7.83 Hz;
(vi) the modulation frequency is within the range of 14.0 to 14.5 Hz;
(vi) the modulation frequency is 14.1 Hz;
(vii) the modulation frequency is within the range of 20.0 to 21.0 Hz;
(viii) the modulation frequency is 20.3 Hz;
(ix) the modulation frequency is within the range of 26.0 to 27.5 Hz; or
(x) the modulation frequency is 26.4 Hz.

12. The system or method according to any one of claims 1 to 11, wherein the carrier frequency is 432 Hz and the modulation frequency is 7.83 Hz.

13. The system or method according to any one of claims 1 to 12, wherein the maximum magnetic field strength is 200 µT;
optionally wherein:
(i) the maximum magnetic field strength is 30 µT; or
(ii) the maximum magnetic field strength is 11 µT.

14. The system or method according to any one of the preceding claims, wherein the modulation frequency is sinusoidal and/or the low frequency magnetic field is not pulsed.

## Patentansprüche

1. Magnetfeldexpositionssystem zum Exponieren von organischen Zellen, Zellgewebe oder zumindest einem Teil eines Subjekts gegenüber einem niederfrequenten Magnetfeld, wobei das System einen Magnetfeldgenerator umfasst, der eine oder mehrere Spulen umfasst, die konfiguriert sind, um ein Magnetfeld zu produzieren, das gemäß einem amplitudenmodulierten Signal mit einer Trägerfrequenz von 360 bis 450 Hz variiert, wobei die Modulationsfrequenz von 0,5 bis 100 Hz ist; und wobei das Magnetfeld eine Feldstärke von 0,5 bis 250 µT aufweist.

2. Magnetfeldexpositionssystem nach Anspruch 1, wobei die eine oder mehreren Spulen konfiguriert sind, um das Subjekt, das gegenüber dem niederfrequenten Magnetfeld zu exponieren ist, zumindest teilweise zu umgeben.

3. Magnetfeldexpositionssystem nach einem der vorhergehenden Ansprüche, wobei das System einen Stuhl oder ein Bett umfasst, der/das konfiguriert ist, sodass zumindest Teil eines Subjekts, das auf dem Stuhl sitzt oder in dem Bett liegt, gegenüber dem niederfrequenten Magnetfeld exponiert ist.

4. Magnetfeldexpositionssystem nach Anspruch 3, wobei der Stuhl einen Sitz (1801) umfasst, der sich in einem Hohlraum befindet, der zwischen zwei Seitenwänden (1802) gebildet ist;
wobei sich optional kreisförmige Spulen (1803) mit dem gleichem Durchmesser in jeder Seitenwand (1802) befinden, wobei sich die Spulen (1803) koaxial befinden, wodurch eine Helmholtz-Spule gebildet wird.

5. Magnetfeldexpositionssystem nach einem der Ansprüche 1 bis 3, wobei das System ein Bett umfasst und die eine oder mehreren Spulen das Bett umgeben oder sich über und/oder unter dem Bett befinden;
wobei optional:
(i) die eine oder mehreren Spulen das Bett umgeben und sich koaxial entlang der horizontalen Achse des Bettes befinden; und/oder
(ii) die eine oder mehreren Spulen eine Helmholtz-Spule umfassen.

6. Magnetfeldexpositionssystem nach Anspruch 1, wobei das System ein Gehäuse umfasst, wobei sich die eine oder mehreren Spulen innerhalb des Gehäuses befinden, wobei das Gehäuse konfiguriert ist, um positioniert zu sein, um einen spezifischen Körperteil oder ein spezifisches Gewebe eines Subjekts gegenüber dem niederfrequenten Magnetfeld zu exponieren;
wobei optional die eine oder mehreren Spulen eine Helmholtz-Spule umfassen.

7. Magnetfeldexpositionssystem nach Anspruch 1, wobei das System in der Form eines Zellkultursystems ist, wobei das System Zellkulturgefäß, das einen Zellkulturhohlraum innerhalb des Gefäßes definiert, und einen Magnetfeldgenerator mit einer oder mehreren Spulen umfasst, die konfiguriert sind, um ein Magnetfeld innerhalb des Zellkulturhohlraums zu produzieren, das gemäß einem amplitudenmodulierten Signal mit einer Trägerfrequenz von 360 bis 450 Hz variiert, wobei die Modulationsfrequenz von 0,5 bis 100 Hz ist; und wobei das Magnetfeld eine Feldstärke von 0,5 bis 250 µT aufweist.

8. *In*-*vitro*-Verfahren zum Steigern von Proliferation von organischen Zellen durch Exponieren der Zellen gegenüber einem niederfrequenten Magnetfeld, das gemäß einem amplitudenmodulierten Signal mit einer Trägerfrequenz von 360 bis 450 Hz variiert, wobei die Modulationsfrequenz von 0,5 bis 100 Hz ist; und wobei das Magnetfeld eine Feldstärke von 0,5 bis 250 µT aufweist;
wobei optional:
(i) die Zellen Stammzellen, Vorläuferzellen oder andere primäre Zellen sind;
(ii) die Zellen Stammzellen oder Vorläuferzellen sind;
(iii) die Zellen menschliche Stammzellen oder menschliche Vorläuferzellen sind;
(iv) die Zellen menschliche Keratinozytenstammzellen oder menschliche Keratinozytenvorläuferzellen sind;
(v) die Zellen terminal differenzierte primäre Zellen sind; oder
(vi) die Zellen adulte menschliche Stammzellen ausgewählt aus Muskelstammzellen, hämatopoetischen Stammzellen, epithelialen Stammzellen, neuralen Stammzellen, mesenchymalen Stammzellen, Bruststammzellen, intestinalen Stammzellen, mesodermalen Stammzellen, endothelialen Stammzellen, Hautstammzellen, Melanozytenstammzellen, Haarfollikelstammzellen, olfaktorischen Stammzellen, Neuralleistenstammzellen und Zahnmarkstammzellen sind.

9. Nicht-therapeutisches Verfahren zum Behandeln eines Zustands assoziiert mit Hautstammzellen oder Hautvorläuferzellen bei einem Subjekt, wobei das Verfahren Exponieren von Zellgewebe von zumindest einem Teil des Subjekts gegenüber einem niederfrequenten Magnetfeld umfasst, das gemäß einem amplitudenmodulierten Signal mit einer Trägerfrequenz von 360 bis 450 Hz variiert, wobei die Modulationsfrequenz von 0,5 bis 100 Hz ist; und wobei das Magnetfeld eine Feldstärke von 0,5 bis 250 µT aufweist;
wobei optional der Zustand assoziiert mit Hautstammzellen oder Hautvorläuferzellen ausgewählt ist aus Aufrechterhalten von Hauthomöostase, Reduzieren von Hautalterung, Steigern von Hautverjüngung, Verhindern oder Reduzieren von Hautfalten, Erhöhen von Hautelastizität, Verhindern oder Reduzieren von Hautdehnungsstreifen, Verhindern oder Reduzieren von Cellulitis, Erhöhen von Hautfeuchtigkeit, Reduzieren von Hautrauheit, Reduzieren von Hautporengröße, Induzieren von Haarwachstum, Verhindern oder Reduzieren von Haarverlust, Hemmen von Ergrauen der Haare, Induzieren von Haarrepigmentierung, Aufrechterhalten von Hautpigmentierung und Induzieren von Nagelwachstum.

10. Nicht-therapeutisches Verfahren zum Verbessern von Wohlbefinden und/oder Steigern von Konzentration bei einem Subjekt durch Exponieren von zumindest einem Teil des Subjekts gegenüber einem niederfrequenten Magnetfeld, das gemäß einem amplitudenmodulierten Signal mit einer Trägerfrequenz von 360 bis 450 Hz variiert, wobei die Modulationsfrequenz von 0,5 bis 100 Hz ist; und wobei das Magnetfeld eine Feldstärke von 0,5 bis 250 µT aufweist;
wobei optional
(i) das Verfahren ein nicht-therapeutisches Verfahren zum Induzieren von Entspannung, Steigern von Konzentration, Erhöhen von Aufmerksamkeit, Erhöhen von Wachsamkeit und/oder Steigern von Kognition ist; und/oder
(ii) das Verfahren Alpha-Band- und/oder Beta-Band-Aktivität bei dem Subjekt erhöht.

11. System oder Verfahren nach einem der Ansprüche 1 bis 10, wobei das Magnetfeld gemäß einem amplitudenmodulierten Signal mit einer Trägerfrequenz von 400 bis 450 Hz und einer Modulationsfrequenz von 3 bis 30 Hz variiert;
wobei optional:
(i) die Trägerfrequenz 432 Hz ist; und/oder
(ii) die Modulationsfrequenz von 3 bis 28 Hz ist;
(iii) die Modulationsfrequenz einem Schumann-Resonanzmodus entspricht;
(iv) die Modulationsfrequenz innerhalb des Bereichs von 7,50 bis 8,00 Hz ist;
(v) die Modulationsfrequenz 7,83 Hz ist;
(vi) die Modulationsfrequenz innerhalb des Bereichs von 14,0 bis 14,5 Hz ist;
(vi) die Modulationsfrequenz 14,1 Hz ist;
(vii) die Modulationsfrequenz innerhalb des Bereichs von 20,0 bis 21,0 Hz ist;
(viii) die Modulationsfrequenz 20,3 Hz ist;
(ix) die Modulationsfrequenz innerhalb des Bereichs von 26,0 bis 27,5 Hz ist; oder
(x) die Modulationsfrequenz 26,4 Hz ist.

12. System oder Verfahren nach einem der Ansprüche 1 bis 11, wobei die Trägerfrequenz 432 Hz ist und die Modulationsfrequenz 7,83 Hz ist.

13. System oder Verfahren nach einem der Ansprüche 1 bis 12, wobei die maximale magnetische Feldstärke 200 µT ist;
wobei optional:
(i) die maximale magnetische Feldstärke 30 µT ist; oder
(ii) die maximale magnetische Feldstärke 11 µT ist.

14. System oder Verfahren nach einem der vorhergehenden Ansprüche, wobei die Modulationsfrequenz sinusförmig ist und/oder das niederfrequente Magnetfeld nicht gepulst ist.

## Revendications

1. Système d'exposition à un champ magnétique permettant d'exposer des cellules organiques, un tissu cellulaire ou au moins une partie d'un sujet à un champ magnétique basse fréquence, ledit système comprenant un générateur de champ magnétique comprenant une ou plusieurs bobines conçues pour produire un champ magnétique qui varie en fonction d'un signal modulé en amplitude avec une fréquence porteuse de 360 à 450 Hz, ladite fréquence de modulation étant de 0,5 à 100 Hz et ledit champ magnétique comportant une intensité de champ de 0,5 à 250 µT.

2. Système d'exposition à un champ magnétique selon la revendication 1, lesdites une ou plusieurs bobines étant conçues pour entourer au moins partiellement le sujet devant être exposé audit champ magnétique basse fréquence.

3. Système d'exposition à un champ magnétique selon l'une quelconque des revendications précédentes, ledit système comprenant une chaise ou un lit conçu de sorte qu'au moins une partie d'un sujet assis sur la chaise ou allongé sur le lit soit exposée au champ magnétique basse fréquence.

4. Système d'exposition à un champ magnétique selon la revendication 3, ladite chaise comprenant un siège (1801) situé dans une cavité formée entre deux parois latérales (1802) ;
éventuellement des bobines circulaires (1803) du même diamètre étant situées dans chaque paroi latérale (1802), lesdites bobines (1803) étant situées co-axialement, formant ainsi une bobine de Helmholtz.

5. Système d'exposition à un champ magnétique selon l'une quelconque des revendications 1 à 3, ledit système comprenant un lit et lesdites une ou plusieurs bobines entourant le lit ou étant situées au-dessus et/ou au-dessous du lit ;
éventuellement :
(i) lesdites une ou plusieurs bobines entourant le lit et étant situées co-axialement le long de l'axe horizontal du lit ; et/ou
(ii) lesdites une ou plusieurs bobines comprenant une bobine de Helmholtz.

6. Système d'exposition à un champ magnétique selon la revendication 1, ledit système comprenant un boîtier avec lesdites une ou plusieurs bobines situées à l'intérieur du boîtier, ledit boîtier étant conçu pour être positionné de manière à exposer une partie du corps ou un tissu spécifique d'un sujet au champ magnétique basse fréquence ;
éventuellement lesdites une ou plusieurs bobines comprenant une bobine de Helmholtz.

7. Système d'exposition à un champ magnétique selon la revendication 1, ledit système se présentant sous la forme d'un système de culture cellulaire, ledit système comprenant un récipient de culture cellulaire définissant une cavité de culture cellulaire à l'intérieur du récipient et un générateur de champ magnétique avec une ou plusieurs bobines conçues pour produire un champ magnétique à l'intérieur de la cavité de culture cellulaire qui varie en fonction d'un signal modulé en amplitude avec une fréquence porteuse de 360 à 450 Hz, ladite fréquence de modulation étant de 0,5 à 100 Hz ; et ledit champ magnétique comportant une intensité de champ de 0,5 à 250 µT.

8. Procédé *in vitro* permettant d'améliorer la prolifération de cellules organiques en exposant les cellules à un champ magnétique basse fréquence qui varie en fonction d'un signal modulé en amplitude avec une fréquence porteuse de 360 à 450 Hz, ladite fréquence de modulation étant de 0,5 à 100 Hz ; et ledit champ magnétique comportant une intensité de champ de 0,5 à 250 µT ;
éventuellement :
(i) lesdites cellules étant des cellules souches, des cellules progénitrices ou d'autres cellules primaires ;
(ii) lesdites cellules étant des cellules souches ou des cellules progénitrices ;
(iii) lesdites cellules étant des cellules souches humaines ou des cellules progénitrices humaines ;
(iv) lesdites cellules étant des cellules souches de kératinocytes humains ou des cellules progénitrices de kératinocytes humains ;
(v) lesdites cellules étant des cellules primaires à différenciation terminale ; ou
(vi) lesdites cellules étant des cellules souches humaines adultes choisies parmi les cellules souches musculaires, les cellules souches hématopoïétiques, les cellules souches épithéliales, les cellules souches neurales, les cellules souches mésenchymateuses, les cellules souches mammaires, les cellules souches intestinales, les cellules souches mésodermiques, les cellules souches endothéliales, les cellules souches cutanées, les cellules souches de mélanocytes, les cellules souches de follicule pileux, les cellules souches olfactives, les cellules souches de crête neurale et les cellules souches de pulpe dentaire.

9. Procédé non thérapeutique de traitement d'un état associé aux cellules souches cutanées ou aux cellules progénitrices cutanées chez un sujet, le procédé comprenant l'exposition d'un tissu cellulaire d'au moins une partie du sujet à un champ magnétique basse fréquence qui varie en fonction d'un signal modulé en amplitude avec une fréquence porteuse de 360 à 450 Hz, ladite fréquence de modulation étant de 0,5 à 100 Hz ; et ledit champ magnétique comportant une intensité de champ de 0,5 à 250 µT ;
éventuellement ledit état associé aux cellules souches cutanées ou aux cellules progénitrices cutanées étant choisi parmi le maintien de l'homéostasie de la peau, la réduction du vieillissement de la peau, l'amélioration du rajeunissement de la peau, la prévention ou la réduction des rides de la peau, l'augmentation de l'élasticité de la peau, la prévention ou la réduction des vergetures de la peau, la prévention ou la réduction de la cellulite, l'augmentation de l'hydratation de la peau, la réduction de la rugosité de la peau, la réduction de la taille des pores de la peau, l'induction de la croissance des cheveux, la prévention ou la réduction de la perte de cheveux, l'inhibition du grisonnement des cheveux, l'induction de la repigmentation des cheveux, le maintien de la pigmentation de la peau et l'induction de la croissance des ongles.

10. Procédé non thérapeutique permettant d'améliorer le bien-être et/ou de renforcer la concentration chez un sujet en exposant au moins une partie du sujet à un champ magnétique basse fréquence qui varie en fonction d'un signal modulé en amplitude avec une fréquence porteuse de 360 à 450 Hz, ladite fréquence de modulation étant de 0,5 à 100 Hz ; et ledit champ magnétique comportant une intensité de champ de 0,5 à 250 µT ;
éventuellement
(i) ledit procédé étant un procédé non thérapeutique permettant d'induire la relaxation, d'améliorer la concentration, d'augmenter la focalisation, d'augmenter la vigilance et/ou d'améliorer la cognition ; et/ou
(ii) ledit procédé augmentant l'activité de la bande alpha et/ou de la bande bêta chez le sujet.

11. Système ou procédé selon l'une quelconque des revendications 1 à 10, ledit champ magnétique variant en fonction d'un signal modulé en amplitude avec une fréquence porteuse de 400 à 450 Hz et une fréquence de modulation de 3 à 30 Hz ;
éventuellement :
(i) ladite fréquence porteuse étant de 432 Hz ; et/ou
(ii) ladite fréquence de modulation étant de 3 à 28 Hz ;
(iii) ladite fréquence de modulation correspondant à un mode de résonance de Schumann ;
(iv) ladite fréquence de modulation se trouvant dans la plage de 7,50 à 8,00 Hz ;
(v) ladite fréquence de modulation étant de 7,83 Hz ;
(vi) ladite fréquence de modulation se trouvant dans la plage de 14,0 à 14,5 Hz ;
(vi) ladite fréquence de modulation étant de 14,1 Hz ;
(vii) ladite fréquence de modulation se trouvant dans la plage de 20,0 à 21,0 Hz ;
(viii) ladite fréquence de modulation étant de 20,3 Hz ;
(ix) ladite fréquence de modulation se trouvant dans la plage de 26,0 à 27,5 Hz ; ou
(x) ladite fréquence de modulation étant de 26,4 Hz.

12. Système ou procédé selon l'une quelconque des revendications 1 à 11, ladite fréquence porteuse étant de 432 Hz et ladite fréquence de modulation étant de 7,83 Hz.

13. Système ou procédé selon l'une quelconque des revendications 1 à 12, ladite intensité maximale du champ magnétique étant de 200 µT ;
éventuellement :
(i) ladite intensité maximale du champ magnétique étant de 30 µT ; ou
(ii) ladite intensité maximale du champ magnétique étant de 11 µT.

14. Système ou procédé selon l'une quelconque des revendications précédentes, ladite fréquence de modulation étant sinusoïdale et/ou ledit champ magnétique basse fréquence n'étant pas pulsé.
